# EUROPÄISCHE PATENTSCHRIFT

(11) **EP 1 430 884 B1**
(45) Veröffentlichungstag und Bekanntmachung des Hinweises auf die Patenterteilung: **02.11.2005**
(21) Anmeldenummer: 03026919.5
(22) Anmeldetag: 25.11.2003
(51) Int. Cl.: A61K 7/50

(54) **Ölduschbad mit spezieller Tensidkombination**
Oil showerbath with a special combination of surfactant
Huile de bain-douche avec une combinaison tensioactive particulière

(30) Priorität: 20.12.2002 DE 10261110
(43) Veröffentlichungstag der Anmeldung: 23.06.2004
(73) Patentinhaber: Hans Schwarzkopf & Henkel GmbH & Co. KG, 22763 Hamburg (DE)
(72) Erfinder: Goddinger, Dieter, Dr., 25336 Klein-Nordende (DE); Retzer, Hedwig, 22529 Hamburg (DE)
(74) Vertreter: Strohe-Kamp, Geertje

(56) Entgegenhaltungen:
- EP-A- 0 691 127
- DE-A- 19 856 555
- US-A- 5 409 640
- WÖRTERBUCH DER KOSMETIK , 1997 4 * Seite 311 *
- RÖMPP CHEMIE LEXIKON , 1995 9 * Seite 5171 *

## Beschreibung

Die Erfindung betrifft Zusammensetzungen auf der Basis einer speziellen Tensidkombination und mindestens eines Ölkörpers, zur besonders milden Reinigung und schonenden Pflege von Haut und Haaren.

Reinigungsmittel für Haut und Haar, wie sie beispielsweise als flüssige Seifen, Shampoos, Duschbäder, Schaumbäder, Dusch- und Waschgele im Handel erhältlich sind, müssen nicht nur ein gutes Reinigungsvermögen aufweisen, sondern sollen weiterhin für die Haut und die Schleimhäute gut verträglich sein und auch bei häufiger Anwendung nicht zu starker Entfettung oder Hauttrockenheit führen. Darüber hinaus beurteilt der Verbraucher die Gebrauchseigenschaften aber auch nach der Menge und Qualität des bei der Anwendung sich bildenden Schaums. Insbesondere wird ein rasches Anschäumen unter Bildung von feinblasigem und beständigem Schaum gewünscht, wobei diese Eigenschaften des Schaums auch meist als die Cremigkeit des Schaums beschrieben werden.

Die Reinigungsmittel für Haut und Haar sollen sich außerdem durch eine gewisse Dickflüssigkeit auszeichnen, so dass sie beispielsweise auf die Hand aufgebracht werden können und nicht, bevor sie auf die Körperoberfläche oder auf dem Kopf verteilt werden, zwischen den Fingern hindurchrinnen.

Es sind zahlreiche hautfreundliche und schleimhautverträgliche Tenside im Stand der Technik bekannt. Die zusätzliche Forderung nach einer gewissen Dickflüssigkeit der wässrigen Lösung und nach einer Feinblasigkeit des Schaums wird jedoch nur von wenigen Tensiden erfüllt. Man hat daher bisher auch stets Kombinationen verschiedener Tenside verwendet, um den vielseitigen Anforderungen gerecht zu werden. So hat sich eine Kombination aus Alkylethersulfat - Tensiden und Alkyl-(poly)-glucosiden als besonders schaumstark und hautverträglich erwiesen.

Beispielsweise ist in der DE-A-42 34 487 eine wässrige Detergenszusammensetzung beschrieben, die Alkylsulfat-Tenside, Alkylethersulfat-Tenside, Alkyl-(oligo)-glucoside und amphotere bzw. zwitterionische Tenside enthält.

Trotz der milden Tensidkombinationen kommt es beim Baden oder Duschen, abhängig von der Dauer und der Temperatur, zu einer Quellung der Hornschicht der Haut, bei der Schmutzbestandteile, aber auch hauteigene Stoffe, die für das Wasserbindungsvermögen der Hornschicht verantwortlich sind, ab- oder ausgewaschen werden können. Zudem können Hautfette in gewissem Maße gelöst und ausgewaschen werden, was nach anfänglicher Quellung eine nachfolgende deutliche Austrocknung zur Folge hat, die durch häufige Behandlung mit waschaktiven Zusätzen noch weiterhin verstärkt werden kann.

Bei gesunder Haut sind diese Vorgänge im allgemeinen nicht von schädlichen Folgen begleitet, da die Schutzmechanismen der Haut solche leichten Störungen der oberen Hautschichten ohne weiteres kompensieren können. Aber durch umweltbedingte Schädigungen, wie beispielsweise Irritationen oder Lichtschäden, ist der Schutzmechanismus der Hautoberfläche gestört und die hauteigenen Schutzmechanismen können diese Störungen nicht alleine kompensieren. In diesen Fällen muss der Schädigung "von außen" entgegengewirkt werden. Zu diesem Zwecke wurden die sogenannten Ölduschbäder entwickelt, die die Rückfettung gewährleisten.

Aus der DE-A1-198 56 555 sind Ölduschbäder bekannt, die eine bestimmte Tensidmischung (50-80%), bestehend aus Fettalkoholpolyglycolethersulfaten, Fettalkoholpolyglycolethern und Alkyl- und/oder Alkenyloligoglycosiden, und 20 bis 50% eines Ölkörpers umfassen.

Die EP 691 127 B1 beschreibt weiterhin die Verwendung anionischer Tenside (55%) und mindestens 45% spezieller Öle mit einem hohen Gehalt an Triglyceriden in wasserfreien Zubereitungen zur Herstellung kosmetischer Duschöle.

Aus der EP 662 815 ist ferner ein Shampoo bekannt, das die Tensidkombination Alkylethersulfat, C₁₂-C₁₄-Fettalkohol+10EO-Essigsäuresalz und C₁₂-C₁₆-Alkylglucosid und 0,8% eines Fettkörpers offenbart.

Es hat sich jedoch herausgestellt, dass in den Produkten der beiden oberen Fälle neben dem hohen Ölkörpergehalt auch ein hoher Tensidgehalt aus anwendungstechnischen Gründen erforderlich ist, was bei dermatologischen Prüfungen zu einer schlechteren Hautverträglichkeit führt, als Produkte, die gar keine Ölkörperkomponente umfassen, aber einen Tensidgehalt von nur ca. 10 bis 15% aufweisen.

Die Produkte der EP 662 815 B1 wiederum weisen einen Ölkörpergehalt auf, der zu gering ist, als dass die oben genannten Nachteile des Standes der Technik damit behoben werden könnten.

Es bestand daher der Bedarf an kosmetischen Zusammensetzungen, die neben einer möglichst hohen Einsatzmenge eines Ölkörpers einen möglichst geringen Tensidanteil aufweisen.

Vollkommen überraschend wurde festgestellt, dass durch die Kombination spezieller Tenside in einem bestimmten Verhältnis mit Ölkörpern die Einsatzmenge der Tenside in Ölduschbädern deutlich verringert werden kann, ohne dass die Reinigungsleistung abnimmt. Gleichzeitig zeichnen sich die Produkte nachweislich gegenüber den Produkten des Standes der Technik (s.o.) durch eine verbesserte Hautverträglichkeit, einen cremigeren und stabileren Schaum und hervorragende Milde aus.

Gegenstand der Erfindung sind daher wässrige Körperreinigungs- und Pflegezusammensetzungen, enthaltend
- maximal 35 Gew.-% einer milden Tensidmischung, bestehend aus
   (A) mindestens einem anionischen Tensid vom Typ der Fettalkoholpolyglycolethersulfate,
   (B) mindestens einem Tensid auf Zuckerbasis und
   (C) mindestens einem Co-Tensid, ausgewählt aus der Gruppe die gebildet wird aus Sulfosuccinaten, Sulfobernsteinsäureestern, α-Olefinsulfonaten, Acyltauriden und Fettalkohol-Ethercarbonsäuren, und
- 2 bis 25 Gew.-% eines Ölkörpers,
mit einem Gewichtsverhältnis der Tenside von (A) : (B) : (C) = 10 : (0,5 - 5) : (0,5 - 5).

Erfindungsgemäß umfassen die wässrigen Körperreinigungs- und Pflegezusammensetzungen bevorzugt 0,1 - 35 Gew.-% der Tensidkomponente, insbesondere 1 - 30 Gew.-%, jeweils bezogen auf das Gesamtgewicht der Zusammensetzungen.

Bevorzugt im Sinne der Erfindung sind wässrige Körperreinigungs- und Pflegezusammensetzungen, die als Komponente (A) Fettalkoholpolyglycolethersulfate der Formel (I)

R¹O-(CH₂CH₂O)ₙSO₃X (I)

in der R1 für einen linearen oder verzweigten Alkyl- und/oder Alkenylrest mit 12 bis 18 Kohlenstoffatomen, n für Zahlen von 1 bis 5 und X für Alkali oder Ammonium steht.

Die Komponenten (A) werden in der Tensidmischung der erfindungsgemäßen Zusammensetzungen in einer Menge von 0,1 bis 80 Gew.-% eingesetzt. Bevorzugt ist eine Einsatzmenge von 10 bis 75 Gew.-% und insbesondere bevorzugt ist eine Einsatzmenge des anionischen Tensids (A) im Bereich von 25-70 Gew.-%.

Als Komponenten (B) im Sinne der Erfindung werden Alkyl- und Alkenyloligoglycoside der Formel (II) eingesetzt,

(II), R² -O-[G]ₘ (II),

wobei R² für Alkyl- und/oder Alkenylrest mit 4 - 22 Kohlenstoffatomen, G für einen Zuckerrest mit 5 - 6 Kohlenstoffatomen und m für Zahlen von 1 - 10 steht.

Die erfindungsgemäß verwendbaren Alkylpolyglykoside können lediglich einen bestimmten Alkylrest R² enthalten. Üblicherweise werden diese Verbindungen aber ausgehen von natürlichen Fetten und Ölen oder Mineralölen hergestellt. In diesem Fall liegen als Alkylreste R² Mischungen entsprechend den Ausgangsverbindungen bzw. entsprechend der jeweiligen Aufarbeitung dieser Verbindungen vor.

Besonders bevorzugt sind solche Alkylpolyglykoside, bei denen R²
- im wesentlichen aus C₈- und C₁₀-Alkylgruppen,
- im wesentlichen aus C₁₂- und C₁₄-Alkylgruppen,
- im wesentlichen aus C₈- bis C₁₆-Alkylgruppen oder
- im wesentlichen aus C₁₂- bis C₁₆-Alkylgruppen oder
- im wesentlichen aus C₁₆ bis C₁₈-Alkylgruppen besteht.

Als Zuckerbaustein Z können beliebige Mono- oder Oligosaccharide eingesetzt werden. Üblicherweise werden Zucker mit 5 bzw. 6 Kohlenstoffatomen sowie die entsprechenden Oligosaccharide eingesetzt. Solche Zucker sind beispielsweise Glucose, Fructose, Galactose, Arabinose, Ribose, Xylose, Lyxose, Allose, Altrose, Mannose, Gulose, Idose, Talose und Sucrose. Bevorzugte Zuckerbausteine sind Glucose, Fructose, Galactose, Arabinose und Sucrose; Glucose ist besonders bevorzugt.
Die erfindungsgemäß verwendbaren Alkylpolyglykoside enthalten im Schnitt 1,1 bis 5 Zuckereinheiten. Alkylpolyglykoside mit m-Werten von 1,1 bis 2,0 sind bevorzugt. Ganz besonders bevorzugt sind Alkylglykoside, bei denen m 1,1 bis 1,8 beträgt.

Auch die alkoxylierten Homologen der genannten Alkylpolyglykoside können erfindungsgemäß eingesetzt werden. Diese Homologen können durchschnittlich bis zu 10 Ethylenoxid- und/oder Propylenoxideinheiten pro Alkylglykosideinheit enthalten.

Die Komponenten (B) werden in der Tensidmischung der erfindungsgemäßen Zusammensetzungen in einer Menge von 0,1 bis 30 Gew.-% eingesetzt. Bevorzugt ist eine Einsatzmenge von 0,5 bis 25 Gew.-% und insbesondere bevorzugt ist eine Einsatzmenge des Tensids auf Zuckerbasis (B) im Bereich von 1-20 Gew.-%.

Bevorzugte Co-Tenside (C) im Sinne der Erfindung sind Fettalkohol-Ethercarbonsäuren der Formel (III)

CH₃(CH₂)ₚ(OCH₂CH₂)_{q}OCH₂(CH₂)ᵣCOOH (III)

sowie physiologisch verträgliche Salze dieser Verbindungen, in denen p steht für Zahlen von 6 bis 30, q steht für Zahlen von 1 bis 30 und r steht für Zahlen von 0 bis 22.

Unter physiologisch verträglichen Salzen im Sinne der Erfindung sind Alkalimetallsalze wie Natrium und Kalium, Erdalkalimetallsalze wie Magnesium und Calcium, Ammonium-, Alkylammonium-, Alkanolammonium- oder Glucammoniumsalze zu verstehen.

Die Co-Tenside (C) werden in der Tensidmischung der erfindungsgemäßen Zusammensetzungen in einer Menge von 0,1 bis 30 Gew.-% eingesetzt. Bevorzugt ist eine Einsatzmenge von 0,5 bis 25 Gew.-% und insbesondere bevorzugt ist eine Einsatzmenge der Co-Tenside (C) im Bereich von 1-20 Gew.-%.

Es kann erfindungsgemäß bevorzugt sein, zusätzliche weitere Tenside aus der Gruppe der anionischen, kationischen, zwitterionischen, amphoteren oder nichtionischen Tenside einzusetzen.

Als weitere anionische Tenside eignen sich in erfindungsgemäßen Zubereitungen alle für die Verwendung am menschlichen Körper geeigneten anionischen oberflächenaktiven Stoffe. Diese sind gekennzeichnet durch eine wasserlöslich machende, anionische Gruppe wie z. B. eine Carboxylat-, Sulfat-, Sulfonat- oder phat-Gruppe und eine lipophile Alkylgruppe mit etwa 8 bis 30 C-Atomen. Zusätzlich können im Molekül Glykol- oder Polyglykolether-Gruppen, Ester-, Ether- und Amidgruppen sowie Hydroxylgruppen enthalten sein. Beispiele für geeignete anionische Tenside sind, jeweils in Form der Natrium-, Kalium- und Ammonium- sowie der Mono-, Di- und Trialkanolammoniumsalze mit 2 bis 4 C-Atomen in der Alkanolgruppe,
- lineare und verzweigte Fettsäuren mit 8 bis 30 C-Atomen (Seifen),
- Acylsarcoside mit 8 bis 24 C-Atomen in der Acylgruppe,
- Acyltauride mit 8 bis 24 C-Atomen in der Acylgruppe,
- Acylisethionate mit 8 bis 24 C-Atomen in der Acylgruppe,
- Sulfobernsteinsäuremono- und -dialkylester mit 8 bis 24 C-Atomen in der Alkylgruppe und Sulfobernsteinsäuremono-alkylpolyoxyethylester mit 8 bis 24 C-Atomen in der Alkylgruppe und 1 bis 6 Oxyethylgruppen,
- lineare Alkansulfonate mit 8 bis 24 C-Atomen,
- lineare Alpha-Olefinsulfonate mit 8 bis 24 C-Atomen,
- Sulfosuccinate der Formel (IV) 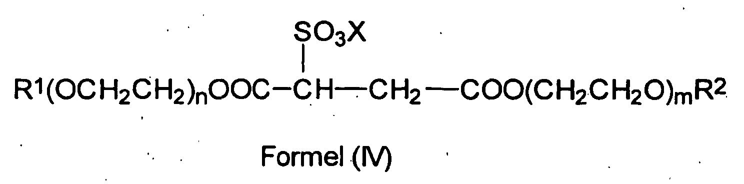 in der R¹ für einen Alkyl- und/oder Alkenylrest mit 6 bis 22 Kohlenstoffatomen, R² für R¹ oder X, m und n unabhängig voneinander für 0 oder Zahlen von 1 bis 10 und X für ein Alkali- oder Erdalkalimetall, Ammonium, Alkylammonium, Alkanolammonium oder Glucammonium steht,
- Alkylsulfate der Formel R-O(CH₂-CH₂O)ₓ-OSO₃H, in der R eine bevorzugt lineare Alkylgruppe mit 8 bis 30 C-Atomen und x = 0 ist,
- Gemische oberflächenaktiver Hydroxysulfonate gemäß DE-A-37 25 030,
- sulfatierte Hydroxyalkylpolyethylen- und/oder Hydroxyalkylenpropylenglykolether gemäß DE-A-37 23 354,
- Sulfonate ungesättigter Fettsäuren mit 8 bis 24 C-Atomen und 1 bis 6 Doppelbindungen gemäß DE-A-39 26 344,
- Ester der Weinsäure und Zitronensäure mit Alkoholen, die Anlagerungsprodukte von etwa 2-15 Molekülen Ethylenoxid und/oder ,Propylenoxid an Fettalkohole mit 8 bis 22 C-Atomen darstellen,
- Alkyl- und/oder Alkenyletherphosphate der Formel (V), 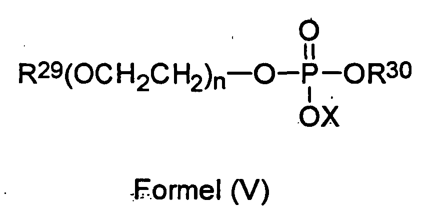 in der R²⁹ bevorzugt für einen aliphatischen Kohlenwasserstoffrest mit 8 bis 30 Kohlenstoffatomen, R³⁰ für Wasserstoff, einen Rest (CH₂CH₂O)ₙR²⁹ oder X, n für Zahlen von 1 bis 10 und X für Wasserstoff, ein Alkali- oder Erdalkalimetall oder NR³¹R³²R³³R³⁴, mit R³¹ bis R³⁴ unabhängig voneinander stehend für einen C₁ bis C₄ - Kohlenwasserstoffrest, steht,
- Monoglyceridsulfate und Monoglyceridethersulfate der Formel (VI) wie sie beispielsweise in der EP-B1 0 561 825, der EP-B1 0 561 999, der DE-A1 42 04 700 oder von A.K.Biswas et al. in J.Am.Oil.Chem.Soc. 37, 171 (1960) und F.U.Ahmed in J.Am.Oil.Chem.Soc. 67, 8 (1990) beschrieben worden sind. 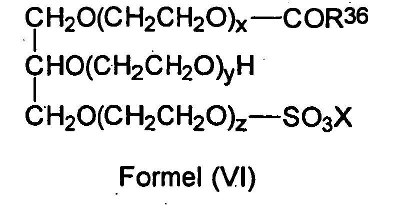 in der R³⁶CO für einen linearen oder verzweigten Acylrest mit 6 bis 22 Kohlenstoffatomen, x, y und z in Summe für 0 oder für Zahlen von 1 bis 30, vorzugsweise 2 bis 10, und X für ein Alkali- oder Erdalkalimetall steht.
- Typische Beispiele für im Sinne der Erfindung geeignete Monoglycerid(ether)sulfate sind die Umsetzungsprodukte von Laurinsäuremonoglycerid, Kokosfettsäuremonoglycerid, Palmitinsäuremonoglycerid, Stearinsäuremonoglycerid, Ölsäuremonoglycerid und Talgfettsäuremonoglycerid sowie deren Ethylenoxidaddukte mit Schwefeltrioxid oder Chlorsulfonsäure in Form ihrer Natriumsalze. Vorzugsweise werden Monoglyceridsulfate der Formel (VIII) eingesetzt, in der R³⁶CO für einen linearen Acylrest mit 8 bis 18 Kohlenstoffatomen steht

Bevorzugte anionische Tenside sind Sulfobernsteinsäuremono- und -dialkylester mit 8 bis 18 C-Atomen in der Alkylgruppe und Sulfobernsteinsäuremonoalkylpolyoxyethylester mit 8 bis 18 C-Atomen in der Alkylgruppe und 1 bis 6 Oxyethylgruppen, Alpha-Olefinsulfonate mit 8 bis 18 C-Atomen, Acyltauride mit 8 bis 18 C-Atomen in der Acylgruppe sowie Sulfosuccinate nach der oben stehenden Formel (IV).

Als zwitterionische Tenside werden solche oberflächenaktiven Verbindungen bezeichnet, die im Molekül mindestens eine quartäre Ammoniumgruppe und mindestens eine -COO⁽⁻⁾ - oder -SO₃⁽⁻⁾ -Gruppe tragen. Besonders geeignete zwitterionische Tenside sind die sogenannten Betaine wie die N-Alkyl-N,N-dimethylammoniumglycinate, beispielsweise das Kokosalkyl-dimethylammoniumglycinat, N-Acylaminopropyl-N,N-dimethylammoniumglycinate, beispielsweise das Kokosacylaminopropyl-dimethylammoniumglycinat, und 2-Alkyl-3-carboxymethyl-3-hydroxyethyl-imidazoline mit jeweils 8 bis 18 C-Atomen in der Alkyl- oder Acylgruppe sowie das Kokosacylaminoethylhydroxyethylcarboxymethylglycinat. Ein bevorzugtes zwitterionisches Tensid ist das unter der INCI-Bezeichnung Cocamidopropyl Betaine bekannte Fettsäureamid-Derivat.

Unter ampholytischen Tensiden werden solche oberflächenaktiven Verbindungen verstanden, die außer einer C₈ - C₂₄ - Alkyl- oder -Acylgruppe im Molekül mindestens eine freie Aminogruppe und mindestens eine -COOH- oder -SO₃H-Gruppe enthalten und zur Ausbildung innerer Salze befähigt sind. Beispiele für geeignete ampholytische Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 24 C-Atomen in der Alkylgruppe. Besonders bevorzugte ampholytische Tenside sind das N-Kokosalkylaminopropionat, das Kokosacylaminoethylaminopropionat und das C₁₂ - C₁₈ - A-cylsarcosin.

Nichtionische Tenside enthalten als hydrophile Gruppe z. B. eine Polyolgruppe, eine Polyalkylenglykolethergruppe oder eine Kombination aus Polyol- und Polyglykolethergruppe. Solche Verbindungen sind beispielsweise
- Anlagerungsprodukte von 2 bis 50 Mol Ethylenoxid und/oder 0 bis 5 Mol Propylenoxid an lineare und verzweigte Fettalkohole mit 8 bis 30 C-Atomen, an Fettsäuren mit 8 bis 30 C-Atomen und an Alkylphenole mit 8 bis 15 C-Atomen in der Alkylgruppe;
- mit einem Methyl- oder C₂-C₆-Alkylrest endgruppenverschlossene Anlagerungsprodukte von 2 bis 50 Mol Ethylenoxid und/oder 0 bis 5 Mol Propylenoxid an lineare und verzweigte Fettalkohole mit 8 bis 30 C-Atomen, an Fettsäuren mit 8 bis 30 C-Atomen und an Alkylphenole mit 8 bis 15 C-Atomen in der Alkylgruppe, wie beispielsweise die unter den Verkaufsbezeichnungen Dehydol® LS, Dehydol® LT (Cognis) erhältlichen Typen,
- C₁₂-C₃₀-Fettsäuremono- und -diester von Anlagerungsprodukten von 1 bis 30 Mol Ethylenoxid an Glycerin,
- Anlagerungsprodukte von 5 bis 60 Mol Ethylenoxid an Rizinusöl und gehärtetes Rizinusöl,
- Polyolfettsäureester, wie beispielsweise das Handelsprodukt Hydagen® HSP (Cognis) oder Sovermol - Typen (Cognis),
- alkoxilierte Triglyceride,
- alkoxilierte Fettsäurealkylester der Formel (VII)

   R³⁷CO-(OCH₂CHR³⁸)_{w}OR³⁹ Formel (VII),

   in der R³⁷CO für einen linearen oder verzweigten, gesättigten und/oder ungesättigten Acylrest mit 6 bis 22 Kohlenstoffatomen, R³⁸ für Wasserstoff oder Methyl, R³⁹ für lineare oder verzweigte Alkylreste mit 1 bis 4 Kohlenstoffatomen und w für Zahlen von 1 bis 20 steht,
- Aminoxide,
- Hydroxymischether, wie sie beipielsweise in der DE-OS 19738866 beschrieben sind,
- Sorbitanfettsäureester und Anlagerungeprodukte von Ethylenoxid an Sorbitanfettsäureester wie beispielsweise die Polysorbate,
- Zuckerfettsäureester und Anlagerungsprodukte von Ethylenoxid an Zuckerfettsäureester,
- Anlagerungsprodukte von Ethylenoxid an Fettsäurealkanolamide und Fettamine,
- Fettsäure-N-alkylglucamide,

Als bevorzugte nichtionische Tenside haben sich die Alkylenoxid-Anlagerungsprodukte an gesättigte lineare Fettalkohole und Fettsäuren mit jeweils 2 bis 30 Mol Ethylenoxid pro Mol Fettalkohol bzw. Fettsäure erwiesen.
Zubereitungen mit hervorragenden Eigenschaften werden ebenfalls erhalten, wenn sie als nichtionische Tenside Fettsäureester von ethoxyliertem Glycerin enthalten.

Diese Verbindungen sind durch die folgenden Parameter gekennzeichnet. Der Alkylrest R enthält 6 bis 22 Kohlenstoffatome und kann sowohl linear als auch verzweigt sein. Bevorzugt sind primäre lineare und in 2-Stellung methylverzweigte aliphatische Reste. Solche Alkylreste sind beispielsweise 1-Octyl, 1-Decyl, 1-Lauryl, 1-Myristyl, 1-Cetyl und 1-Stearyl. Besonders bevorzugt sind 1-Octyl, 1-Decyl, 1-Lauryl, 1-Myristyl. Bei Verwendung sogenannter "Oxo-Alkohole" als Ausgangsstoffe überwiegen Verbindungen mit einer ungeraden Anzahl von Kohlenstoffatomen in der Alkylkette.

Bei den als Tensid eingesetzten Verbindungen mit Alkylgruppen kann es sich jeweils um einheitliche Substanzen handeln. Es ist jedoch in der Regel bevorzugt, bei der Herstellung dieser Stoffe von nativen pflanzlichen oder tierischen Rohstoffen auszugehen, so daß man Substanzgemische mit unterschiedlichen, vom jeweiligen Rohstoff abhängigen Alkylkettenlängen erhält.

Bei den Tensiden, die Anlagerungsprodukte von Ethylen- und/oder Propylenoxid an Fettalkohole oder Derivate dieser Anlagerungsprodukte darstellen, können sowohl Produkte mit einer "normalen" Homologenverteilung als auch solche mit einer eingeengten Homologenverteilung verwendet werden. Unter "normaler" Homologenverteilung werden dabei Mischungen von Homologen verstanden, die man bei der Umsetzung von Fettalkohol und Alkylenoxid unter Verwendung von Alkalimetallen, Alkalimetallhydroxiden oder Alkalimetallalkoholaten als Katalysatoren erhält. Eingeengte Homologenverteilungen werden dagegen erhalten, wenn beispielsweise Hydrotalcite, Erdalkalimetallsalze von Ethercarbonsäuren, Erdalkalimetalloxide, -hydroxide oder -alkoholate als Katalysatoren verwendet werden. Die Verwendung von Produkten mit eingeengter Homologenverteilung kann bevorzugt sein.

Die zusätzlichen Tenside werden in Mengen von 0,1 - 45 Gew.%, bevorzugt 1 - 30 Gew.% und ganz besonders bevorzugt von 1 - 15 Gew.%, bezogen auf das gesamte Mittel, eingesetzt.

In einer bevorzugten Ausführungsform können nicht-ionische, zwitterionische und/oder amphotere Tenside sowie deren Mischungen bevorzugt sein.

Erfindungsgemäß bevorzugt sind ebenfalls kationische Tenside vom Typ der quartären Ammoniumverbindungen, der Esterquats und der Amidoamine. Bevorzugte quaternäre Ammoniumverbindungen sind Ammoniumhalogenide, insbesondere Chloride und Bromide, wie Alkyltrimethylammoniumchloride, Dialkyldimethylammoniumchloride und Trialkylmethylammoniumchloride, z. B. Cetyltrimethylammoniumchlorid, Stearyltrimethylammoniumchlorid, Distearyldimethylammoniumchlorid, Lauryldimethylammoniumchlorid, Lauryldimethylbenzylammoniumchlorid und Tricetylmethylammoniumchlorid, sowie die unter den INCI-Bezeichnungen Quaternium-27 und Quaternium-83 bekannten Imidazolium-Verbindungen. Die langen Alkylketten der oben genannten Tenside weisen bevorzugt 10 bis 18 Kohlenstoffatome auf.

Bei Esterquats handelt es sich um bekannte Stoffe, die sowohl mindestens eine Esterfunktion als auch mindestens eine quartäre Ammoniumgruppe als Strukturelement enthalten. Bevorzugte Esterquats sind quaternierte Estersalze von Fettsäuren mit Triethanolamin, quaternierte Estersalze von Fettsäuren mit Diethanolalkylaminen und quaternierten Estersalzen von Fettsäuren mit 1,2-Dihydroxypropyldialkylaminen. Solche Produkte werden beispielsweise unter den Warenzeichen Stepantex®, Dehyquart® und Armocare® vertrieben. Die Produkte Armocare® VGH-70, ein N,N-Bis(2-Palmitoyloxyethyl)dimethylammoniumchlorid, sowie Dehyquart® F-75, Dehyquart® C-4046, Dehyquart® L80 und Dehyquart® AU-35 sind Beispiele für solche Esterquats.

Die Alkylamidoamine werden üblicherweise durch Amidierung natürlicher oder synthetischer Fettsäuren und Fettsäureschnitte mit Dialkylaminoaminen hergestellt. Eine erfindungsgemäß besonders geeignete Verbindung aus dieser Substanzgruppe stellt das unter der Bezeichnung Tegoamid® S 18 im Handel erhältliche Stearamidopropyl-dimethylamin dar.

Die kationischen Tenside sind in den erfindungsgemäß verwendeten Mitteln bevorzugt in Mengen von 0,05 bis 10 Gew.-%, bezogen auf das gesamte Mittel, enthalten. Mengen von 0,1 bis 5 Gew.-% sind besonders bevorzugt.

In einer weiteren bevorzugten Ausführungsform kann die Wirkung des erfindungsgemäßen Wirkstoffes durch Emulgatoren gesteigert werden. Solche Emulgatoren sind beispielsweise
- Anlagerungsprodukte von 4 bis 30 Mol Ethylenoxid und/oder 0 bis 5 Mol Propylenoxid an lineare Fettalkohole mit 8 bis 22 C-Atomen, an Fettsäuren mit 12 bis 22 C-Atomen und an Alkylphenole mit 8 bis 15 C-Atomen in der Alkylgruppe,
- C₁₂-C₂₂-Fettsäuremono- und -diester von Anlagerungsprodukten von 1 bis 30 Mol Ethylenoxid an Polyole mit 3 bis 6 Kohlenstoffatomen, insbesondere an Glycerin,
- Ethylenoxid- und Polyglycerin-Anlagerungsprodukte an Methylglucosid-Fettsäureester, Fettsäurealkanolamide und Fettsäureglucamide,
- C₈-C₂₂-Alkylmono- und -oligoglycoside und deren ethoxylierte Analoga, wobei Oligomerisierungsgrade von 1,1 bis 5, insbesondere 1,2 bis 2,0, und Glucose als Zuckerkomponente bevorzugt sind,
- Gemische aus Alkyl-(oligo)-glucosiden und Fettalkoholen zum Beispiel das im Handel erhältliche Produkt Montanov®68,
- Anlagerungsprodukte von 5 bis 60 Mol Ethylenoxid an Rizinusöl und gehärtetes Rizinusöl,
- Partialester von Polyolen mit 3-6 Kohlenstoffatomen mit gesättigten Fettsäuren mit 8 bis 22 C-Atomen,
- Sterine. Als Sterine wird eine Gruppe von Steroiden verstanden, die am C-Atom 3 des Steroid-Gerüstes eine Hydroxylgruppe tragen und sowohl aus tierischem Gewebe (Zoosterine) wie auch aus pflanzlichen Fetten (Phytosterine) isoliert werden. Beispiele für Zoosterine sind das Cholesterin und das Lanosterin. Beispiele geeigneter Phytosterine sind Ergosterin, Stigmasterin und Sitosterin. Auch aus Pilzen und Hefen werden Sterine, die sogenannten Mykosterine, isoliert.
- Phospholipide. Hierunter werden vor allem die Glucose-Phospolipide, die z.B. als Lecithine bzw. Phospahtidylcholine aus z.B. Eidotter oder Pflanzensamen (z.B. Sojabohnen) gewonnen werden, verstanden.
- Fettsäureester von Zuckern und Zuckeralkoholen, wie Sorbit, Polyglycerine und Polyglycerinderivate wie beispielsweise Polyglycerinpoly-12-hydroxystearat (Handelsprodukt Dehymuls® PGPH)
- Lineare und verzweigte Fettsäuren mit 8 bis 30 C - Atomen und deren Na-, K-, Ammonium-, Ca-, Mg- und Zn - Salze.

Die erfindungsgemäßen Mittel enthalten die Emulgatoren bevorzugt in Mengen von 0,1 - 25 Gew.-%, insbesondere 0,5 - 15 Gew.-%, bezogen auf das gesamte Mittel.

Bevorzugt können die erfindungsgemäßen Zusammensetzungen mindestens einen nichtionogenen Emulgator mit einem HLB-Wert von 8 bis 18, gemäß den im Römpp-Lexikon Chemie (Hrg. J. Falbe, M.Regitz), 10. Auflage, Georg Thieme Verlag Stuttgart, New York, (1997), Seite 1764, aufgeführten Definitionen enthalten. Nichtionogene Emulgatoren mit einem HLB-Wert von 10 - 15 können erfindungsgemäß besonders bevorzugt sein.

Unter den genannten Emulgatoren-Typen können die Emulgatoren, welche kein Ethylenoxid und/oder Propylenoxid im Molekül enthalten ganz besonders bevorzugt sein.

Ein weiterer wesentlicher Bestandteil der Erfindung ist ein Ölkörper und/oder Fettstoff. Unter Ölkörpern und/oder Fettstoffen sind zu verstehen Fettsäuren, Fettalkohole, natürliche und synthetische Wachse, welche sowohl in fester Form als auch flüssig in wäßriger Dispersion vorliegen können, sowie natürliche und synthetische kosmetische Ölkomponenten.

Als Fettsäuren können eingesetzt werden lineare und/oder verzweigte, gesättigte und/oder ungesättigte Fettsäuren mit 6 - 30 Kohlenstoffatomen. Bevorzugt sind Fettsäuren mit 10 - 22 Kohlenstoffatomen. Hierunter wären beispielsweise zu nennen die Isostearinsäuren, wie die Handelsprodukte Emersol® 871 und Emersol® 875, und Isopalmitinsäuren wie das Handelsprodukt Edenor® IP 95, sowie alle weiteren unter den Handelsbezeichnungen Edenor® (Cognis) vertriebenen Fettsäuren. Weitere typische Beispiele für solche Fettsäuren sind Capronsäure, Caprylsäure, 2-Ethylhexansäure, Caprinsäure, Laurinsäure, Isotridecansäure, Myristinsäure, Palmitinsäure, Palmitoleinsäure, Stearinsäure, Isostearinsäure, Ölsäure, Elaidinsäure, Petroselinsäure, Linolsäure, Linolensäure, Elaeostearinsäure, Arachinsäure, Gadoleinsäure, Behensäure und Erucasäure sowie deren technische Mischungen, die z.B. bei der Druckspaltung von natürlichen Fetten und Ölen, bei der Oxidation von Aldehyden aus der Roelen'schen Oxosynthese oder der Dimerisierung von ungesättigten Fettsäuren anfallen. Besonders bevorzugt sind üblicherweise die Fettsäureschnitte, welche aus Cocosöl oder Palmöl erhältlich sind; insbesondere bevorzugt ist in der Regel der Einsatz von Stearinsäure.

Die Einsatzmenge beträgt dabei 0,1 - 15 Gew.%, bezogen auf das gesamte Mittel. In einer bevorzugten Ausführungsform beträgt die Menge 0,5 - 10 Gew.%, wobei ganz besonders vorteilhaft Mengen von 1 - 5 Gew.% sind.

Als Fettalkohole können eingesetzt werden gesättigte, ein- oder mehrfach ungesättigte, verzweigte oder unverzweigte Fettalkohole mit C₆ - C₃₀-, bevorzugt C₁₀ - C₂₂- und ganz besonders bevorzugt C₁₂ - C₂₂- Kohlenstoffatomen. Einsetzbar im Sinne der Erfindung sind beispielsweise Decanol, Octanol, Octenol, Dodecenol, Decenol, Octadienol, Dodecadienol, Decadienol, Oleylalkohol, Erucaalkohol, Ricinolalkohol, Stearylalkohol, Isostearylalkohol, Cetylalkohol, Laurylalkohol, Myristylalkohol, Arachidylalkohol, Caprylalkohol, Caprinalkohol, Linoleylalkohol, Linolenylalkohol und Behenylalkohol, sowie deren Guerbetalkohole, wobei diese Aufzählung beispielhaften und nicht limitierenden Charakter haben soll. Die Fettalkohole stammen jedoch von bevorzugt natürlichen Fettsäuren ab, wobei üblicherweise von einer Gewinnung aus den Estern der Fettsäuren durch Reduktion ausgegangen werden kann. Erfindungsgemäß einsetzbar sind ebenfalls solche Fettalkoholschnitte, die durch Reduktion natürlich vorkommender Triglyceride wie Rindertalg, Palmöl, Erdnußöl, Rüböl, Baumwollsaatöl, Sojaöl, Sonnenblumenöl und Leinöl oder aus deren Umesterungsprodukten mit entsprechenden Alkoholen entstehenden Fettsäureestern erzeugt werden, und somit ein Gemisch von unterschiedlichen Fettalkoholen darstellen. Solche Substanzen sind beispielsweise unter den Bezeichnungen Stenol®, z.B. Stenol® 1618 oder Lanette®, z.B. Lanette® O oder Lorol®, z.B. Lorol® C8, Lorol® C14, Lorol® C18, Lorol® C8-18, HD-Ocenol®, Crodacol®, z.B. Crodacol® CS, Novol®, Eutanol® G, Guerbitol® 16, Guerbitol® 18, Guerbitol® 20, Isofol® 12, Isofol® 16, Isofol® 24, Isofol® 36, Isocarb® 12, Isocarb® 16 oder Isocarb® 24 käuflich zu erwerben. Selbstverständlich können erfindungsgemäß auch Wollwachsalkohole, wie sie beispielsweise unter den Bezeichnungen Corona®, White Swan®, Coronet® oder Fluilan® käuflich zu erwerben sind, eingesetzt werden. Die Fettalkohole werden in Mengen von 0,1 - 20 Gew.-%, bezogen auf die gesamte Zubereitung, bevorzugt in Mengen von 0,1 - 10 Gew.-% eingesetzt.

Als natürliche oder synthetische Wachse können erfindungsgemäß eingesetzt werden feste Paraffine oder Isoparaffine, Carnaubawachse, Bienenwachse, Candelillawachse, Ozokerite, Ceresin, Walrat, Sonnenblumenwachs, Fruchtwachse wie beispielsweise Apfelwachs oder Citruswachs, Microwachse aus PE- oder PP. Derartige Wachse sind beispielsweise erhältlich über die Fa. Kahl & Co., Trittau.

Zu den natürlichen und synthetischen kosmetischen Ölkörpern, welche die Wirkung des erfindungsgemäßen Wirkstoffes steigern können, sind beispielsweise zu zählen:
- pflanzliche Öle. Beispiele für solche Öle sind Sonnenblumenöl, Olivenöl, Sojaöl, Rapsöl, Mandelöl, Jojobaöl, Orangenöl, Weizenkeimöl, Pfirsichkernöl und die flüssigen Anteile des Kokosöls. Geeignet sind aber auch andere Triglyceridöle wie die flüssigen Anteile des Rindertalgs sowie synthetische Triglyceridöle,
- flüssige Paraffinöle, Isoparaffinöle und synthetische Kohlenwasserstoffe sowie Di-n-alkylether mit insgesamt zwischen 12 bis 36 C-Atomen, insbesondere 12 bis 24 C-Atomen, wie beispielsweise Di-n-octylether, Di-n-decylether, Di-n-nonylether, Di-n-undecylether, Di-n-dodecylether, n-Hexyl-n-octylether, n-Octyl-n-decylether, n-Decyl-n-undecylether, n-Undecyl-n-dodecylether und n-Hexyl-n-Undecylether sowie Di-tert-butylether, Di-iso-pentylether, Di-3-ethyldecylether, tert.-Butyl-n-octylether, iso-Pentyl-n-octylether und 2-Methylpentyl-n-octylether. Die als Handelsprodukte erhältlichen Verbindungen 1,3-Di-(2-ethyl-hexyl)-cyclohexan (Cetiol® S) und Di-n-octylether (Cetiol® OE) können bevorzugt sein,
- Esteröle. Unter Esterölen sind zu verstehen die Ester von C₆ - C₃₀ - Fettsäuren mit C₂ - C₃₀ - Fettalkoholen. Bevorzugt sind die Monoester der Fettsäuren mit Alkoholen mit 2 bis 24 C-Atomen. Beispiele für eingesetzte Fettsäurenanteile in den Estern sind Capronsäure, Caprylsäure, 2-Ethylhexansäure, Caprinsäure, Laurinsäure, Isotridecansäure, Myristinsäure, Palmitinsäure, Palmitoleinsäure, Stearinsäure, Isostearinsäure, Ölsäure, Elaidinsäure, Petroselinsäure, Linolsäure, Linolensäure, Elaeostearinsäure, Arachinsäure, Gadoleinsäure, Behensäure und Erucasäure sowie deren technische Mischungen, die z.B. bei der Druckspaltung von natürlichen Fetten und Ölen, bei der Oxidation von Aldehyden aus der Roelen'schen Oxosynthese oder der Dimerisierung von ungesättigten Fettsäuren anfallen. Beispiele für die Fettalkoholanteile in den Esterölen sind Isopropylalkohol, Capronalkohol, Caprylalkohol, 2-Ethylhexylalkohol, Caprinalkohol, Laurylalkohol, Isotridecylalkohol, Myristylalkohol, Cetylalkohol, Palmoleylalkohol, Stearylalkohol, Isostearylalkohol, Oleylalkohol, Elaidylalkohol, Petroselinylalkohol, Linolylalkohol, Linolenylalkohol, Elaeostearylalkohol, Arachylalkohol, Gadoleylalkohol, Behenylalkohol, E-rucylalkohol und Brassidylalkohol sowie deren technische Mischungen, die z.B. bei der Hochdruckhydrierung von technischen Methylestern auf Basis von Fetten und Ölen oder Aldehyden aus der Roelen'schen Oxosynthese sowie als Monomerfraktion bei der Dimerisierung von ungesättigten Fettalkoholen anfallen. Erfindungsgemäß besonders bevorzugt sind Isopropylmyristat (Rilanit® IPM), Isononansäure-C16-18-alkylester (Cetiol® SN), 2-Ethylhexylpalmitat (Cegesoft® 24), Stearinsäure-2-ethylhexylester (Cetiol® 868), Cetyloleat, Glycerintricaprylat, Kokosfettalkohol-caprinat/-caprylat (Cetiol® LC), n-Butylstearat, Oleylerucat (Cetiol® J 600), Isopropylpalmitat (Rilanit® IPP), Oleyl Oleate (Cetiol®), Laurinsäurehexylester (Cetiol® A), Di-n-butyladipat (Cetiol® B), Myristylmyristat (Cetiol®, MM), Cetearyl Isononanoate (Cetiol® SN), Ölsäuredecylester (Cetiol® V),
- Dicarbonsäureester wie Di-n-butyladipat, Di-(2-ethylhexyl)-adipat, Di-(2-ethylhexyl)-succinat und Di-isotridecylacelaat sowie Diolester wie Ethylenglykol-dioleat, Ethylenglykol-di-isotridecanoat, Propylenglykol-di(2-ethylhexanoat), Propylenglykol-di-isostearat, Propylenglykol-di-pelargonat, Butandiol-di-isostearat, Neopentylglykoldicaprylat,
- symmetrische, unsymmetrische oder cyclische Ester der Kohlensäure mit Fettalkoholen, beispielsweise beschrieben in der DE-OS 197 56 454, Glycerincarbonat oder Dicaprylylcarbonat (Cetiol® CC),
- Mono,- Di- und Trifettsäureester von gesättigten und/oder ungesättigten linearen und/oder verzweigten Fettsäuren mit Glycerin, wie beispielsweise Monomuls® 90-018, Monomuls® 90-L12 oder Cutina® MD.

Erfindungsgemäß ganz besonders bevorzugt sind die Ölkörper aus der Gruppe, die zusammengesetzt ist aus Esterölen von C₆-C₃₀-Fettsäuren mit C₂-C₃₀-Fettalkoholen, Fettsäureglyceride aus Mono-, Di- und Trifettsäureestern von ge-Erfindungsgemäß ganz besonders bevorzugt sind die Ölkörper aus der Gruppe, die zusammengesetzt ist aus Esterölen von C₆-C₃₀-Fettsäuren mit C₂-C₃₀-Fettalkoholen, Fettsäureglyceride aus Mono-, Di- und Trifettsäureestern von gesättigten und/oder ungesättigten linearen und/oder verzweigten C₆-C₃₀-Fettsäuren mit Glycerin sowie ethoxylierte Fettsäureglyceride aus Mono-, Di- und Trifettsäureestern von gesättigten und/oder ungesättigten linearen und/oder verzweigten C₆-C₃₀-Fettsäuren mit Glycerin. Bevorzugt ist ein Ethoxylierungsgrad von 2 bis 20.

Die Gesamtmenge der Öl- und Fettkomponenten in den erfindungsgemäßen Mitteln beträgt üblicherweise 2 - 25 Gew.-%, bezogen auf das gesamte Mittel. Mengen von 4 - 25 Gew.-% sind erfindungsgemäß bevorzugt und Mengen von 6 bis 20 Gew.-% sind erfindungsgemäß besonders bevorzugt.

In einer bevorzugten Ausführungsform der erfindungsgemäßen Lehre kann die Wirkung mit kationischen und/oder nichtionischen Polymeren noch weiter gesteigert werden.

Unter kationischen Polymeren sind Polymere zu verstehen, welche in der Haupt- und/oder Seitenkette Gruppen aufweisen, welche "temporär" oder "permanent" kationisch sein kann. Als "permanent kationisch" werden erfindungsgemäß solche Polymere bezeichnet, die unabhängig vom pH-Wert des Mittels eine kationische Gruppe aufweisen. Dies sind in der Regel Polymere, die ein quartäres Stickstoffatom, beispielsweise in Form einer Ammoniumgruppe, enthalten. Bevorzugte kationische Gruppen sind quartäre Ammoniumgruppen. Insbesondere solche Polymere, bei denen die quartäre Ammoniumgruppe über eine C₁₋₄-Kohlenwasserstoffgruppe an eine aus Acrylsäure, Methacrylsäure oder deren Derivaten aufgebaute Polymerhauptkette gebunden sind, haben sich als besonders geeignet erwiesen.
- Homopolymere der allgemeinen Formel (VIII), 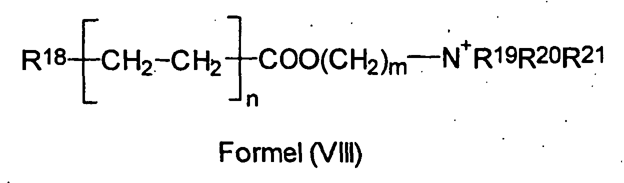 in der R¹⁸ = -H oder -CH₃ ist, R¹⁹, R²⁰ und R²¹ unabhängig voneinander ausgewählt sind aus C₁₋₄-Alkyl-, -Alkenyl- oder -Hydroxyalkylgruppen, m = 1, 2, 3 oder 4, n eine natürliche Zahl und X- ein physiologisch verträgliches organisches oder anorganisches Anion ist, sowie Copolymere, bestehend im wesentlichen aus den in Formel (VIII) aufgeführten Monomereinheiten sowie nichttionogenen Monomereinheiten, sind besonders bevorzugte kationische Polymere. Im Rahmen dieser Polymere sind diejenigen erfindungsgemäß bevorzugt, für die mindestens eine der folgenden Bedingungen gilt:
   - R¹⁸ steht für eine Methylgruppe
   - R¹⁹, R²⁰ und R²¹ stehen für Methylgruppen
   - m hat den Wert 2.
   Als physiologisch verträgliches Gegenionen X- kommen beispielsweise Halogenidionen, Sulfationen, Phosphationen, Methosulfationen sowie organische Ionen wie Lactat-, Citrat-, Tartrat- und Acetationen in Betracht. Bevorzugt sind Halogenidionen, insbesondere Chlorid.
   Ein besonders geeignetes Homopolymer ist das, gewünschtenfalls vernetzte,
   Poly(methacryloyloxyethyltrimethylammoniumchlorid) mit der INCI-Bezeichnung Polyquaternium-37. Die Vernetzung kann gewünschtenfalls mit Hilfe mehrfach olefinisch ungesättigter Verbindungen, beispielsweise Divinylbenzol, Tetraallyloxyethan, Methylenbisacrylamid, Diallylether, Polyallylpolyglycerylether, oder Allylethern von Zuckern oder Zuckerderivaten wie Erythritol, Pentaerythritol, Arabitol, Mannitol, Sorbitol, Sucrose oder Glucose erfolgen. Methylenbisacrylamid ist ein bevorzugtes Vernetzungsagens.
   Das Homopolymer wird bevorzugt in Form einer nichtwäßrigen Polymerdispersion, die einen Polymeranteil nicht unter 30 Gew.-% aufweisen sollte, eingesetzt. Solche Polymerdispersionen sind unter den Bezeichnungen Salcare® SC 95 (ca. 50 % Polymeranteil, weitere Komponenten: Mineralöl (INCI-Bezeichnung: Mineral Oil) und Tridecyl-polyoxypropylen-polyoxyethylen-ether (INCI-Bezeichnung: PPG-1-Trideceth-6)) und Salcare® SC 96 (ca. 50 % Polymeranteil, weitere Komponenten: Mischung von Diestern des Propylenglykols mit einer Mischung aus Capryl- und Caprinsäure (INCl-Bezeichnung: Propylene Glycol Dicaprylate/Dicaprate) und Tridecyl-polyoxypropylen-polyoxyethylen-ether (INCI-Bezeichnung: PPG-1-Trideceth-6)) im Handel erhältlich.
   Copolymere mit Monomereinheiten gemäß Formel (III) enthalten als nichtionogene Monomereinheiten bevorzugt Acrylamid, Methacrylamid, Acrylsäure-C₁₋₄ alkylester und Methacrylsäure-C₁₋₄-alkylester. Unter diesen nichtionogenen Monomeren ist das Acrylamid besonders bevorzugt. Auch diese Copolymere können, wie im Falle der Homopolymere oben beschrieben, vernetzt sein. Ein erfindungsgemäß bevorzugtes Copolymer ist das vernetzte Acrylamid-Methacryloyloxyethyltrimethylammoniumchlorid-Copolymer. Solche Copolymere, bei denen die Monomere in einem Gewichtsverhältnis von etwa 20:80 vorliegen, sind im Handel als ca. 50 %ige nichtwäßrige Polymerdispersion unter der Bezeichnung Salcare® SC 92 erhältlich.
- quaternisierte Cellulose-Derivate, wie sie unter den Bezeichnungen Celquat® und Polymer JR® im Handel erhältlich sind. Die Verbindungen Celquat® H 100, Celquat® L 200 und Polymer JR®400 sind bevorzugte quaternierte Cellulose-Derivate,
- kationische Alkylpolyglycoside gemäß der DE-PS 44 13 686,
- kationiserter Honig, beispielsweise das Handelsprodukt Honeyquat® 50,
- kationische Guar-Derivate, wie insbesondere die unter den Handelsnamen Cosmedia®Guar und Jaguar® vertriebenen Produkte,
- Polysiloxane mit quaternären Gruppen, wie beispielsweise die im Handel erhältlichen Produkte Q2-7224 (Hersteller: Dow Corning; ein stabilisiertes Trimethylsilylamodimethicon), Dow Corning® 929 Emulsion (enthaltend ein hydroxylamino-modifiziertes Silicon, das auch als Amodimethicone bezeichnet wird), SM-2059 (Hersteller: General Electric), SLM-55067 (Hersteller: Wacker) sowie Abil®-Quat 3270 und 3272 (Hersteller: Th. Goldschmidt; diquaternäre Polydimethylsiloxane, Quaternium-80),
- polymere Dimethyldiallylammoniumsalze und deren Copolymere mit Estern und Amiden von Acrylsäure und Methacrylsäure. Die unter den Bezeichnungen Merquat®100 (Poly(dimethyldiallylammoniumchlorid)) und Merquat®550 (Dimethyldiallylammoniumchlorid-Acrylamid-Copolymer) im Handel erhältlichen Produkte sind Beispiele für solche kationischen Polymere,
- Copolymere des Vinylpyrrolidons mit quaternierten Derivaten des Dialkylaminoalkylacrylats und -methacrylats, wie beispielsweise mit Diethylsulfat quaternierte Vinylpyrrolidon-Dimethylaminoethylmethacrylat-Copolymere. Solche Verbindungen sind unter den Bezeichnungen Gafquat®734 und Gafquat® 755 im Handel erhältlich,
- Vinylpyrrolidon-Vinylimidazoliummethochlorid-Copolymere, wie sie unter den Bezeichnungen Luviquat® FC 370, FC 550, FC 905 und HM 552 angeboten werden.
- quaternierter Polyvinylalkohol, sowie die unter den Bezeichnungen
   Polyquaternium 2,
   Polyquaternium 17,
   Polyquaternium 18 und
   Polyquaternium 27 bekannten Polymeren mit quartären Stickstoffatomen in der Polymerhauptkette.

Gleichfalls als kationische Polymere eingesetzt werden können die unter den Bezeichnungen Polyquaternium-24 (Handelsprodukt z. B. Quatrisoft® LM 200), bekannten Polymere. Ebenfalls erfindungsgemäß verwendbar sind die Copolymere des Vinylpyrrolidons, wie sie als Handelsprodukte Copolymer 845 (Hersteller: ISP), Gaffix® VC 713 (Hersteller: ISP), Gafquat®ASCP 1011, Gafquat®HS 110, Luviquat®8155 und Luviquat® MS 370 erhältlich sind.

Weitere erfindungsgemäße kationische Polymere sind die sogenannten "temporär kationischen" Polymere. Diese Polymere enthalten üblicherweise eine Aminogruppe, die bei bestimmten pH-Werten als quartäre Ammoniumgruppe und somit kationisch vorliegt. Bevorzugt sind beispielsweise Chitosan und dessen Derivate, wie sie beispielsweise unter den Handelsbezeichnungen Hydagen® CMF, Hydagen® HCMF, Kytamer® PC und Chitolam® NB/101 im Handel frei verfügbar sind. Chitosane sind deacetylierte Chitine, die in unterschiedlichen Deacetylierungsgraden und unterschiedlichen Abbaugraden (Molekulargewichten) im Handel erhältlich sind. Ihre Herstellung ist z.B. in DE 44 40 625 A1 und in DE 1 95 03 465 A1 beschrieben.

Besonders gut geeignete Chitosane weisen einen Deacetylierungsgrad von wenigstens 80 % und ein Molekulargewicht von 5 · 10⁵ bis 5 · 10⁶ (g/mol) auf.

Zur Herstellung erfindungsgemäßer Zubereitungen muß das Chitosan in die Salzform überführt werden. Dies kann durch Auflösen in verdünnten wäßrigen Säuren erfolgen. Als Säuren sind sowohl Mineralsäuren wie z.B. Salzsäure, Schwefelsäure und Phosphorsäure als auch organische Säuren, z.B. niedermolekulare Carbonsäuren, Polycarbonsäuren und Hydroxycarbonsäuren geeignet. Weiterhin können auch höhermolekulare Alkylsulfonsäuren oder Alkylschwefelsäuren oder Organophosphorsäuren verwendet werden, soweit diese die erforderliche physiologische Verträglichkeit aufweisen. Geeignete Säuren zur Überführung des Chitosans in die Salzform sind z.B. Essigsäure, Glycolsäure, Weinsäure, Apfelsäure, Citronensäure, Milchsäure, 2-Pyrrolidinon-5-carbonsäure, Benzoesäure oder Salicylsäure. Bevorzugt werden niedermolekulare Hydroxycarbonsäuren wie z.B. Glycolsäure oder Milchsäure verwendet.

Insbesondere im Sinne der Erfindung bevorzugte kationischen Polymere sind kationische Cellulosederivate, Homopolymere des Dimethyldiallylammonium-chlorids oder Copolymere des Dimethyldiallylammoniumchlorids mit Acrylamid und kationische Guar-Gumme.

Geeignete nichtionogene Polymere sind beispielsweise:
- Vinylpyrrolidon/Vinylester-Copolymere, wie sie beispielsweise unter dem Warenzeichen Luviskol® (BASF) vertrieben werden. Luviskol® VA 64 und Luviskol® VA 73, jeweils Vinylpyrrolidon/Vinylacetat-Copolymere, sind ebenfalls bevorzugte nichtionische Polymere,
- Celluloseether, wie Hydroxypropylcellulose, Hydroxyethylcellulose und Methylhydroxypropylcellulose, wie sie beispielsweise unter den Warenzeichen Culminal® und Benecel® (AQUALON) vertrieben werden,
- Schellack
- Polyvinylpyrrolidone, wie sie beispielsweise unter der Bezeichnung Luviskol® (BASF) vertrieben werden,
- Siloxane. Diese Siloxane können sowohl wasserlöslich als auch wasserunlöslich sein. Geeignet sind sowohl flüchtige als auch nichtflüchtige Siloxane, wobei als nichtflüchtige Siloxane solche Verbindungen verstanden werden, deren Siedepunkt bei Normaldruck oberhalb von 200 °C liegt. Bevorzugte Siloxane sind Polydialkylsiloxane, wie beispielsweise Polydimethylsiloxan, Polyalkylarylsiloxane, wie beispielsweise Polyphenylmethylsiloxan, ethoxylierte Polydialkylsiloxane sowie Polydialkylsiloxane, die Amin- und/oder Hydroxy-Gruppen enthalten,
- Glycosidisch substituierte Silicone gemäß der EP 0612759 B1,
- Copolymere des Polyethylens oder Polypropylens mit Terephthalsäure, wie sie beispielsweise unter der Bezeichnnung Aristoflex® PEA im Handel vertrieben werden.

Die kationischen und/oder nichtionischen Polymere in den erfindungsgemäßen Zusammensetzungen bevorzugt in einer Menge von 0,01 bis 10 Gew.-% enthalten.

Es kann erfindungsgemäß bevorzugt sein, den Zusammensetzungen zusätzlich zu den kationischen und/oder nichtionischen Polymeren weiterhin anionische und/oder amphotere Polymere zuzusetzen.

Bei den anionischen Polymeren, welche die Wirkung des erfindungsgemäßen Wirkstoffes unterstützen können, handelt es sich um ein anionische Polymere, welche Carboxylat- und/oder Sulfonatgruppen aufweisen. Beispiele für anionische Monomere, aus denen derartige Polymere bestehen können, sind Acrylsäure, Methacrylsäure, Crotonsäure, Maleinsäureanhydrid und 2-Acrylamido-2-methylpropansulfonsäure. Dabei können die sauren Gruppen ganz oder teilweise als Natrium-, Kalium-, Ammonium-, Mono- oder Triethanolammonium-Salz vorliegen. Bevorzugte Monomere sind 2-Acrylamido-2-methylpropansulfonsäure und Acrylsäure.

Als ganz besonders wirkungsvoll haben sich anionische Polymere erwiesen, die als alleiniges oder Co-Monomer 2-Acrylamido-2-methylpropansulfonsäure enthalten, wobei die Sulfonsäuregruppe ganz oder teilweise als Natrium-, Kalium-, Ammonium-, Mono- oder Triethanolammonium-Salz vorliegen kann.

Besonders bevorzugt ist das Homopolymer der 2-Acrylamido-2-methylpropansulfonsäure, das beispielsweise unter der Bezeichnung Rheothik®11-80 im Handel erhältlich ist.

Innerhalb dieser Ausführungsform kann es bevorzugt sein, Copolymere aus mindestens einem anionischen Monomer und mindestens einem nichtionogenen Monomer einzusetzen. Bezüglich der anionischen Monomere wird auf die oben aufgeführten Substanzen verwiesen. Bevorzugte nichtionogene Monomere sind Acrylamid, Methacrylamid, Acrylsäureester, Methacrylsäureester, Vinylpyrrolidon, Vinylether und Vinylester.

Bevorzugte anionische Copolymere sind Acrylsäure-Acrylamid-Copolymere sowie insbesondere Polyacrylamidcopolymere mit Sulfonsäuregruppen-haltigen Monomeren. Ein besonders bevorzugtes anionisches Copolymer besteht aus 70 bis 55 Mol-% Acrylamid und 30 bis 45 Mol-% 2-Acrylamido-2-methylpropansulfonsäure, wobei die Sulfonsäuregruppe ganz oder teilweise als Natrium-, Kalium-, Ammonium-, Mono- oder Triethanolammonium-Salz vorliegt. Dieses Copolymer kann auch vernetzt vorliegen, wobei als Vernetzungsagentien bevorzugt polyolefinisch ungesättigte Verbindungen wie Tetraallyloxyethan, Allylsucrose, Allylpentaerythrit und Methylen-bisacrylamid zum Einsatz kommen. Ein solches Polymer ist in dem Handelsprodukt Sepigel®305 der Firma SEPPIC enthalten. Die Verwendung dieses Compounds, das neben der Polymerkomponente eine Kohlenwasserstoffmischung (C₁₃-C₁₄-Isoparaffin) und einen nichtionogenen Emulgator (Laureth-7) enthält, hat sich im Rahmen der erfindungsgemäßen Lehre als besonders vorteilhaft erwiesen.

Auch die unter der Bezeichnung Simulgel®600 als Compound mit Isohexadecan und Polysorbat-80 vertriebenen Natriumacryloyldimethyltaurat-Copolymere haben sich als erfindungsgemäß besonders wirksam erwiesen.

Ebenfalls bevorzugte anionische Homopolymere sind unvernetzte und vernetzte Polyacrylsäuren. Dabei können Allylether von Pentaerythrit, von Sucrose und von Propylen bevorzugte Vernetzungsagentien sein. Solche Verbindungen sind beispielsweise unter dem Warenzeichen Carbopol® im Handel erhältlich.

Copolymere aus Maleinsäureanhydrid und Methylvinylether, insbesondere solche mit Vernetzungen, sind ebenfalls farberhaltende Polymere. Ein mit 1,9-Decadiene vernetztes Maleinsäure-Methylvinylether-Copolymer ist unter der Bezeichnungg Stabileze® QM im Handel erhältlich.

Weiterhin können als Polymere zur Steigerung der Wirkung des erfindungsgemäßen Wirkstoffes amphotere Polymere als Bestandteil eingesetzt. Unter dem Begriff amphotere Polymere werden sowohl solche Polymere, die im Molekül sowohl freie Aminogruppen als auch freie -COOH- oder SO₃H-Gruppen enthalten und zur Ausbildung innerer Salze befähigt sind, als auch zwitterionische Polymere, die im Molekül quartäre Ammoniumgruppen und -COO⁻- oder -SO₃⁻-Gruppen enthalten, und solche Polymere zusammengefaßt, die -COOH- oder SO₃H-Gruppen und quartäre Ammoniumgruppen enthalten.

Ein Beispiel für ein erfindungsgemäß einsetzbares Amphopolymer ist das unter der Bezeichnung Amphomer® erhältliche Acrylharz, das ein Copolymeres aus tert.-Butylaminoethylmethacrylat, N-(1, 1,3,3-Tetramethylbutyl)acrylamid sowie zwei oder mehr Monomeren aus der Gruppe Acrylsäure, Methacrylsäure und deren einfachen Estern darstellt.

Weitere erfindungsgemäß einsetzbare amphotere Polymere sind die in der britischen Offenlegungsschrift 2 104 091, der europäischen Offenlegungsschrift 47 714, der europäischen Offenlegungsschrift 217 274, der europäischen Offenlegungsschrift 283 817 und der deutschen Offenlegungsschrift 28 17 369 genannten Verbindungen.

Bevorzugt eingesetzte amphotere Polymere sind solche Polymerisate, die sich im wesentlichen zusammensetzen aus
(a) Monomeren mit quartären Ammoniumgruppen der allgemeinen Formel (IX),

   R²²-CH=CR²³-CO-Z-(CₙH₂ₙ)-N⁽⁺⁾R²⁴R²⁵R²⁶ A⁽⁻⁾ (IX)

   in der R²² und R²³ unabhängig voneinander stehen für Wasserstoff oder eine Methylgruppe und R²⁴, R²⁵ und R²⁶ unabhängig voneinander für Alkylgruppen mit 1 bis 4 Kohlenstoff-Atomen, Z eine NH-Gruppe oder ein Sauerstoffatom, n eine ganze Zahl von 2 bis 5 und A⁽⁻⁾ das Anion einer organischen oder anorganischen Säure ist und
(b) monomeren Carbonsäuren der allgemeinen Formel (X),

   R²⁷-CH=CR²⁸-COOH (X)

   in denen R²⁷ und R²⁸ unabhängig voneinander Wasserstoff oder Methylgruppen sind.

Diese Verbindungen können sowohl direkt als auch in Salzform, die durch Neutralisation der Polymerisate, beispielsweise mit einem Alkalihydroxid, erhalten wird, erfindungsgemäß eingesetzt werden. Bezüglich der Einzelheiten der Herstellung dieser Polymerisate wird ausdrücklich auf den Inhalt der deutschen Offenlegungsschrift 39 29 973 Bezug genommen. Ganz besonders bevorzugt sind solche Polymerisate, bei denen Monomere des Typs (a) eingesetzt werden, bei denen R²⁴, R²⁵ und R²⁶ Methylgruppen sind, Z eine NH-Gruppe und A⁽⁻⁾ ein Halogenid-, Methoxysulfat- oder Ethoxysulfat-Ion ist; Acrylamidopropyl-trimethylammoniumchlorid ist ein besonders bevorzugtes Monomeres (a). Als Monomeres (b) für die genannten Polymerisate wird bevorzugt Acrylsäure verwendet.

Unter dem Begriff Polymer sind erfindungsgemäß ebenfalls spezielle Zubereitungen von Polymeren wie sphärische Polymerpulver zu verstehen. Es sind verschiedene Verfahren bekannt, solche Mikrokugeln aus verschiedenen Monomeren herzustellen, z.B. durch spezielle Polymerisationsverfahren oder durch Auflösen des Polymeren in einem Lösungsmittel und Versprühen in ein Medium, in dem das Lösungsmittel verdunsten oder aus den Teilchen herausdiffundieren kann. Ein solches Verfahren ist z.B. aus EP 466 986 B1 bekannt. Geeignete Polymerisate sind z.B. Polycarbonate, Polyurethane, Polyacrylate, Polyolefine, Polyester oder Polyamide. Besonders geeignet sind solche sphärischen Polymerpulver, deren Primärpartikeldurchmesser unter 1 µm liegt. Solche Produkte auf Basis eines Polymethacrylat-Copolymers sind z.B. unter dem Warenzeichen Polytrap®Q5-6603 (Dow Corning) im Handel. Andere Polymerpulver, z.B. auf Basis von Polyamiden (Nylon 6, Nylon 12) sind mit einer Teilchengröße von 2 - 10 µm (90 %) und einer spezifischen Oberfläche von ca. 10 m²/g unter der Handelsbezeichnung Orgasol® 2002 DU Nat Cos (Atochem S.A., Paris) erhältlich. Weitere sphärische Polymerpulver, die für den erfindungsgemäßen Zweck geeignet sind, sind z.B. die Polymethacrylate (Micropearl M) von SEPPIC oder (Plastic Powder A) von NIKKOL, die Styrol-Divinylbenzol-Copolymeren (Plastic Powder FP) von NIKKOL, die Polyethylen- und Polypropylen-Pulver (ACCUREL EP 400) von AKZO, oder auch Silikonpolymere (Silicone Powder X2-1605) von Dow Corning oder auch sphärische Cellulosepulver.

Die anionischen und/oder amphoteren Polymere sind in den erfindungsgemäß verwendeten Mitteln bevorzugt in Mengen von 0,01 bis 5 Gew.-%, bezogen auf das gesamte Mittel, enthalten. Mengen von 0,01 bis 5, insbesondere von 0,1 bis 3 Gew.-%, sind besonders bevorzugt.

In einer bevorzugten Ausführungsform der Erfindung enthalten die Zusammensetzungen weiterhin mindestens einen Vertreter aus der Gruppe der Polyole.

Polyole, die im Sinne der Erfindung in Betracht kommen, besitzen vorzugsweise 2 bis 15 Kohlenstoffatome und mindestens zwei Hydroxylgruppen. Typische Beispiele sind
- Glycerin;
- Alkylenglycole, wie beispielsweise Ethylenglycol, Diethylenglycol, Propylenglycol, Butylenglycol, Hexylenglycol sowie Polyethylenglycole mit einem durchschnittlichen Molekulargewicht von 100 bis 1.000 Dalton;
- technische Oligoglyceringemische mit einem Eigenkondensationsgrad von 1,5 bis 10 wie etwa technische Diglyceringemische mit einem Diglyceringehalt von 40 bis 50 Gew.-%;
- Methyolverbindungen, wie insbesondere Trimethylolethan, Trimethylolpropan, Trimethylolbutan, Pentaerythrit und Dipentaerythrit;
- Niedrigalkylglucoside, insbesondere solche, mit 1 bis 8 Kohlenstoffen im Alkylrest, wie beispiels-weise Methyl- und Butylglucosid;
- Zuckeralkohole mit 5 bis 12 Kohlenstoffatomen, wie beispielsweise Sorbit oder Mannit,
- Zucker mit 5 bis 12 Kohlenstoffatomen, wie beispielsweise Glucose oder Saccharose;
- Aminozucker, wie beispielsweise Glucamin,
- Pentan-Dodecandiole.

Erfindungsgemäß bevorzugt sind Zusammensetzungen mit einem Gemisch aus mehreren Polyolen. Insbesondere bevorzugt ist ein Gemisch aus Glycerin, Sorbit, 1,2-Propylenglycol und Polyethylenglycol.

Das Polyolgemisch wird in den erfindungsgemäßen Zusammensetzungen bevorzugt in Mengen von 0,1 bis 35 Gew.-% eingesetzt, insbesondere bevorzugt ist eine Einsatzmenge im Bereich von 1 bis 20 Gew.-%.

Weiterhin können in den erfindungsgemäß verwendeten Mitteln Proteinhydrolysate und/oder Aminosäuren und deren Derivate (H) enthalten sein. Proteinhydrolysate sind Produktgemische, die durch sauer, basisch oder enzymatisch katalysierten Abbau von Proteinen (Eiweißen) erhalten werden. Unter dem Begriff Proteinhydrolysate werden erfindungsgemäß auch Totalhydrolysate sowie einzelne Aminosäuren und deren Derivate sowie Gemische aus verschiedenen Aminosäuren verstanden. Weiterhin werden erfindungsgemäß aus Aminosäuren und Aminosäurederivaten aufgebaute Polymere unter dem Begriff Proteinhydrolysate verstanden. Zu letzteren sind beispielsweise Polyalanin, Polyasparagin, Polyserin etc. zu zählen. Weitere Beispiele für erfindungsgemäß einsetzbare Verbindungen sind L-Alanyl-L-prolin, Polyglycin, Glycyl-L-glutamin oder D/L-Methionin-S-Methylsulfoniumchlorid. Selbstverständlich können erfindungsgemäß auch β-Aminosäuren und deren Derivate wie β-Alanin, Anthranilsäure oder Hippursäure eingesetzt werden. Das Molgeweicht der erfindungsgemäß einsetzbaren Proteinhydrolysate liegt zwischen 75, dem Molgewicht für Glycin, und 200000, bevorzugt beträgt das Molgewicht 75 bis 50000 und ganz besonders bevorzugt 75 bis 20000 Dalton.

Erfindungsgemäß können Proteinhydrolysate sowohl pflanzlichen als auch tierischen oder marinen oder synthetischen Ursprungs eingesetzt werden.

Tierische Proteinhydrolysate sind beispielsweise Elastin-, Kollagen-, Keratin-, Seiden- und Milcheiweiß-Proteinhydrolysate, die auch in Form von Salzen vorliegen können. Solche Produkte werden beispielsweise unter den Warenzeichen Dehylan® (Cognis), Promois® (Interorgana), Collapuron® (Cognis), Nutrilan® (Cognis), Gelita-Sol® (Deutsche Gelatine Fabriken Stoess & Co), Lexein® (Inolex) und Kerasol® (Croda) vertrieben.

Erfindungsgemäß bevorzugt ist die Verwendung von Proteinhydrolysaten pflanzlichen Ursprungs, z. B. Soja-, Mandel-, Erbsen-, Kartoffel- und Weizenproteinhydrolysate. Solche Produkte sind beispielsweise unter den Warenzeichen Gluadin® (Cognis), DiaMin® (Diamalt), Lexein® (Inolex), Hydrosoy® (Croda), Hydrolupin® (Croda), Hydrosesame® (Croda), Hydrotritium® (Croda) und Crotein® (Croda) erhältlich.

Wenngleich der Einsatz der Proteinhydrolysate als solche bevorzugt ist, können an deren Stelle gegebenenfalls auch anderweitig erhaltene Aminosäuregemische eingesetzt werden. Ebenfalls möglich ist der Einsatz von Derivaten der Proteinhydrolysate, beispielsweise in Form ihrer Fettsäure-Kondensationsprodukte Solche Produkte werden beispielsweise unter den Bezeichnungen Lamepon® (Cognis), Lexein® (Inolex), Crolastin® (Croda) oder Crotein® (Croda) vertrieben.

Die Proteinhydrolysate oder deren Derivate sind in den erfindungsgemäß verwendeten Zubereitungen bevorzugt in Mengen von 0,1 bis 10 Gew.-%, bezogen auf das gesamte Mittel, enthalten. Mengen von 0,1 bis 5 Gew.-% sind besonders bevorzugt.

Weiterhin können 2-Pyrrolidinon-5-carbonsäure und/oder deren Derivate (J) in den Zubereitungen des erfindungsgemäßen Verfahrens verwendet werden. Bevorzugt sind die Natrium-, Kalium-, Calcium-, Magnesium- oder Ammoniumsalze, bei denen das Ammoniumion neben Wasserstoff eine bis drei C₁- bis C₄-Alkylgruppen trägt. Das Natriumsalz ist ganz besonders bevorzugt. Die eingesetzten Mengen in den erfindungsgemäßen Mitteln betragen 0,01 bis 10 Gew.%, bezogen auf das gesamte Mittel, besonders bevorzugt 0,1 bis 5, und insbesondere 0,1 bis 3 Gew.%.

Ebenfalls als vorteilhaft hat sich die Verwendung von Vitaminen, Provitaminen und Vitaminvorstufen sowie deren Derivaten (K) erwiesen.

Dabei sind erfindungsgemäß solche Vitamine, Pro-Vitamine und Vitaminvorstufen bevorzugt, die üblicherweise den Gruppen A, B, C, E, F und H zugeordnet werden.

Zur Gruppe der als Vitamin A bezeichneten Substanzen gehören das Retinol (Vitamin A₁) sowie das 3,4-Didehydroretinol (Vitamin A₂). Das β-Carotin ist das Provitamin des Retinols. Als Vitamin A-Komponente kommen erfindungsgemäß beispielsweise Vitamin A-Säure und deren Ester, Vitamin A-Aldehyd und Vitamin A-Alkohol sowie dessen Ester wie das Palmitat und das Acetat in Betracht. Die erfindungsgemäß verwendeten Zubereitungen enthalten die Vitamin A-Komponente bevorzugt in Mengen von 0,05-1 Gew.-%, bezogen auf die gesamte Zubereitung.

Zur Vitamin B-Gruppe oder zu dem Vitamin B-Komplex gehören u. a.
Vitamin B₁ (Thiamin)
Vitamin B₂ (Riboflavin)
Vitamin B₃ Unter dieser Bezeichnung werden häufig die Verbindungen Nicotinsäure und Nicotinsäureamid (Niacinamid) geführt. Erfindungsgemäß bevorzugt ist das Nicotinsäureamid, das in den erfindungsgemäß verwendetenen Mitteln bevorzugt in Mengen von 0,05 bis 1 Gew.-%, bezogen auf das gesamte Mittel, enthalten ist.
Vitamin B₅ (Pantothensäure, Panthenol und Pantolacton). Im Rahmen dieser Gruppe wird bevorzugt das Panthenol und/oder Pantolacton eingesetzt. Erfindungsgemäß einsetzbare Derivate des Panthenols sind insbesondere die Ester und Ether des Panthenols sowie kationisch derivatisierte Panthenole. Einzelne Vertreter sind beispielsweise das Panthenoltriacetat, der Panthenolmonoethylether und dessen Monoacetat sowie die in der WO 92/13829 offenbarten kationischen Panthenolderivate. Die genannten Verbindungen des Vitamin B₅-Typs sind in den erfindungsgemäß verwendeten Mitteln bevorzugt in Mengen von 0,05 - 10 Gew.-%, bezogen auf das gesamte Mittel, enthalten. Mengen von 0,1 - 5 Gew.-% sind besonders bevorzugt.
Vitamin B₆ (Pyridoxin sowie Pyridoxamin und Pyridoxal).

Vitamin C (Ascorbinsäure). Vitamin C wird in den erfindungsgemäß verwendeten Mitteln bevorzugt in Mengen von 0,1 bis 3 Gew.-%, bezogen auf das gesamte Mittel eingesetzt. Die Verwendung in Form des Palmitinsäureesters, der Glucoside oder Phosphate kann bevorzugt sein. Die Verwendung in Kombination mit Tocopherolen kann ebenfalls bevorzugt sein.

Vitamin E (Tocopherole, insbesondere α-Tocopherol). Tocopherol und seine Derivate, worunter insbesondere die Ester wie das Acetat; das Nicotinat, das Phosphat und das Succinat fallen, sind in den erfindungsgemäß verwendeten Mitteln bevorzugt in Mengen von 0,05-1 Gew.-%, bezogen auf das gesamte Mittel, enthalten.

Vitamin F. Unter dem Begriff "Vitamin F" werden üblicherweise essentielle Fettsäuren, insbesondere Linolsäure, Linolensäure und Arachidonsäure, verstanden.

Vitamin H. Als Vitamin H wird die Verbindung (3a*S*,4*S*, 6a*R*)-2-Oxohexahydrothienol[3,4-*d*]-imidazol-4-valeriansäure bezeichnet, für die sich aber inzwischen der Trivialname Biotin durchgesetzt hat. Biotin ist in den erfindungsgemäß verwendeten Mitteln bevorzugt in Mengen von 0,0001 bis 1,0 Gew.-%, insbesondere in Mengen von 0,001 bis 0,01 Gew.-% enthalten.

Bevorzugt enthalten die erfindungsgemäß verwendeten Zubereitungen Vitamine, Provitamine und Vitaminvorstufen aus den Gruppen A, B, E und H. Selbstverständlich können auch mehrere Vitamine und Vitaminvorstufen gleichzeitig enthalten sein.

Panthenol, Pantolacton, Pyridoxin und seine Derivate sowie Nicotinsäureamid und Biotin sind besonders bevorzugt.

Die Einsatzmenge der Vitamine und Vitaminvorstufen in den erfindungsgemäß verwendeten Mitteln beträgt 0,0001 - 10 Gew.%, bezogen auf das gesamte Mittel, bevorzugt 0,0001 - 5 Gew.-%, und insbesondere 0,0001 - 3 Gew.-%.

Schließlich können in den erfindungsgemäßen Mitteln Pflanzenextrakte (L) verwendet werden.

Üblicherweise werden diese Extrakte durch Extraktion der gesamten Pflanze hergestellt. Es kann aber in einzelnen Fällen auch bevorzugt sein, die Extrakte ausschließlich aus Blüten und/oder Blättern der Pflanze herzustellen.

Hinsichtlich der erfindungsgemäß verwendbaren Pflanzenextrakte wird insbesondere auf die Extrakte hingewiesen, die in der auf Seite 44 der 3. Auflage des Leitfadens zur Inhaltsstoffdeklaration kosmetischer Mittel, herausgegeben vom Industrieverband Körperpflege- und Waschmittel e.V. (IKW), Frankfurt, beginnenden Tabelle aufgeführt sind.

Erfindungsgemäß sind vor allem die Extrakte aus Grünem Tee, Eichenrinde, Brennessel, Hamamelis, Hopfen, Henna, Kamille, Klettenwurzel, Schachtelhalm, Weißdorn, Lindenblüten, Mandel, Aloe Vera, Fichtennadel, Roßkastanie, Sandelholz, Wacholder, Kokosnuß, Mango, Aprikose, Limone, Weizen, Kiwi, Melone, Orange, Grapefruit, Salbei, Rosmarin, Birke, Malve, Wiesenschaumkraut, Quendel, Schafgarbe, Thymian, Melisse, Hauhechel, Huflattich, Eibisch, Meristem, Ginseng und Ingwerwurzel bevorzugt.

Besonders bevorzugt sind die Extrakte aus Grünem Tee, Eichenrinde, Brennessel, Hamamelis, Hopfen, Kamille, Klettenwurzel, Schachtelhalm, Lindenblüten, Mandel, Aloe Vera, Kokosnuß, Mango, Aprikose, Limone, Weizen, Kiwi, Melone, Orange, Grapefruit, Salbei, Rosmarin, Birke, Wiesenschaumkraut, Quendel, Schafgarbe, Hauhechel, Meristem, Ginseng und Ingwerwurzel

Ganz besonders für die erfindungsgemäße Verwendung geeignet sind die Extrakte aus Grünem Tee, Mandel, Aloe Vera, Kokosnuß, Mango, Aprikose, Limone, Weizen, Kiwi und Melone.

Als Extraktionsmittel zur Herstellung der genannten Pflanzenextrakte können Wasser, Alkohole sowie deren Mischungen verwendet werden. Unter den Alkoholen sind dabei niedere Alkohole wie Ethanol und Isopropanol, insbesondere aber mehrwertige Alkohole wie Ethylenglykol und Propylenglykol, sowohl als alleiniges Extraktionsmittel als auch in Mischung mit Wasser, bevorzugt. Pflanzenextrakte auf Basis von Wasser/Propylenglykol im Verhältnis 1:10 bis 10:1 haben sich als besonders geeignet erwiesen.

Die Pflanzenextrakte können erfindungsgemäß sowohl in reiner als auch in verdünnter Form eingesetzt werden. Sofern sie in verdünnter Form eingesetzt werden, enthalten sie üblicherweise ca. 2 - 80 Gew.-% Aktivsubstanz und als Lösungsmittel das bei ihrer Gewinnung eingesetzte Extraktionsmittel oder Extraktionsmittelgemisch.

Weiterhin kann es bevorzugt sein, in den erfindungsgemäßen Mitteln Mischungen aus mehreren, insbesondere aus zwei, verschiedenen Pflanzenextrakten einzusetzen.

Die Einsatzmenge der Pflanzenextrakte in den erfindungsgemäß verwendeten Mitteln beträgt üblicherweise 0,01 - 50 Gew.%, bezogen auf das gesamte Mittel, bevorzugt 0,1 - 30 Gew.-%, und insbesondere 0,1 - 20 Gew.-%.

Vorteilhaft im Sinne der Erfindung können zusätzlich kurzkettige Carbonsäuren (N) eingesetzt werden. Unter kurzkettigen Carbonsäuren und deren Derivaten im Sinne der Erfindung werden Carbonsäuren verstanden, welche gesättigt oder ungesättigt und/oder geradkettig oder verzweigt oder cyclisch und/oder aromatisch und/oder heterocyclisch sein können und ein Molekulargewicht kleiner 750 aufweisen. Bevorzugt im Sinne der Erfindung können gesättigte oder ungesättigte geradkettigte oder verzweigte Carbonsäuren mit einer Kettenlänge von 1 bis zu 16 C-Atomen in der Kette sein, ganz besonders bevorzugt sind solche mit einer Kettenlänge von 1 bis zu 12 C - Atomen in der Kette.

Die kurzkettigen Carbonsäuren im Sinne der Erfindung können ein, zwei, drei oder mehr Carboxygruppen aufweisen. Bevorzugt im Sinne der Erfindung sind Carbonsäuren mit mehreren Carboxygruppen, insbesondere Di- und Tricarbonsäuren. Die Carboxygruppen können ganz oder teilweise als Ester, Säureanhydrid, Lacton, Amid, Imidsäure, Lactam, Lactim, Dicarboximid, Carbohydrazid, Hydrazon, Hydroxam, Hydroxim, Amidin, Amidoxim, Nitril, Phosphon- oder Phosphatester vorliegen. Die erfindungsgemäßen Carbonsäuren können selbstverständlich entlang der Kohlenstoffkette oder des Ringgerüstes substituiert sein. Zu den Substituenten der erfindungsgemäßen Carbonsäuren sind beispielsweise zu zählen C1-C8-Alkyl-, C2-C8-Alkenyl-, Aryl-, Aralkyl- und Aralkenyl-, Hydroxymethyl-, C2-C8-Hydroxyalkyl-,C2-C8-Hydroxyalkenyl-, Aminomethyl-, C2-C8-Aminoalkyl-, Cyano-, Formyl-, Oxo-, Thioxo-, Hydroxy-, Mercapto-, Amino-, Carboxy- oder Iminogruppen. Bevorzugte Substituenten sind C1-C8-Alkyl-, Hydroxymethyl-, Hydroxy-, Amino- und Carboxygruppen. Besonders bevorzugt sind Substituenten in α - Stellung. Ganz besonders bevorzugte Substituenten sind Hydroxy-, Alkoxy- und Aminogruppen, wobei die Aminofunktion gegebenenfalls durch Alkyl-, Aryl-, Aralkyl- und/oder Alkenylreste weiter substituiert sein kann. Weiterhin sind ebenfalls bevorzugte Carbonsäurederivate die Phosphon- und Phosphatester.

Als Beispiele für erfindungsgemäße Carbonsäuren seien genannt Ameisensäure, Essigsäure, Propionsäure, Buttersäure, Isobuttersäure, Valeriansäure, Isovateriansäure, Pivalinsäure, Oxalsäure, Malonsäure, Bernsteinsäure, Glutarsäure, Glycerinsäure, Glyoxylsäure, Adipinsäure, Pimelinsäure, Korksäure, Azelainsäure, Sebacinsäure, Propiolsäure, Crotonsäure, Isocrotonsäure, Elaidinsäure, Maleinsäure, Fumarsäure, Muconsäure, Citraconsäure, Mesaconsäure, Camphersäure, Benzoesäure, o,m,p-Phthalsäure, Naphthoesäure, Toluoylsäure, Hydratropasäure, Atropasäure, Zimtsäure, Isonicotinsäure, Nicotinsäure, Bicarbaminsäure, 4,4'-Dicyano-6,6'-binicotinsäure, 8-Carbamoyloctansäure, 1,2,4-Pentantricarbonsäure, 2-Pyrrolcarbonsäure, 1,2,4,6,7-Napthalinpentaessigsäure, Malonaldehydsäure, 4-Hydroxy-phthalamidsäure, 1-Pyrazolcarbonsäure, Gallussäure oder Propantricarbonsäure, eine Dicarbonsäure ausgewählt aus der Gruppe, die gebildet wird durch Verbindungen der allgemeinen Formel (N-I), 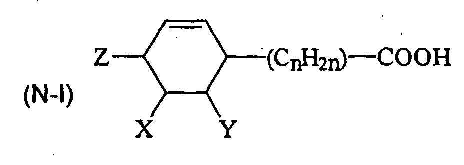 in der Z steht für eine lineare oder verzweigte Alkyl- oder Alkenylgruppe mit 4 bis 12 Kohlenstoffatomen, n für eine Zahl von 4 bis 12 sowie eine der beiden Gruppen X und Y für eine COOH-Gruppe und die andere für Wasserstoff oder einen Methyl- oder Ethylrest, Dicarbonsäuren der allgemeinen Formel (N-I), die zusätzlich noch 1 bis 3 Methyl- oder Ethylsubstituenten am Cyclohexenring tragen sowie Dicarbonsäuren, die aus den Dicarbonsäuren gemäß Formel (N-I) formal durch Anlagerung eines Moleküls Wasser an die Doppelbindung im Cyclohexenring entstehen.

Dicarbonsäuren der Formel (N-I) sind in der Literatur bekannt.

Ein Herstellungsverfahren ist beispielsweise der US-Patentschrift 3,753,968 zu entnehmen.

Die Dicarbonsäuren der Formel (N-I) können beispielsweise durch Umsetzung von mehrfach ungesättigten Dicarbonsäuren mit ungesättigten Monocarbonsäuren in Form einer Diels-Alder-Cyclisierung hergestellt werden. Üblicherweise wird man von einer mehrfach ungesättigten Fettsäure als Dicarbonsäurekomponente ausgehen. Bevorzugt ist die aus natürlichen Fetten und Ölen zugängliche Linolsäure. Als Monocarbonsäurekomponente sind insbesondere Acrylsäure, aber auch z.B. Methacrylsäure und Crotonsäure bevorzugt. Üblicherweise entstehen bei Reaktionen nach Diels-Alder Isomerengemische, bei denen eine Komponente im Überschuß vorliegt. Diese Isomerengemische können erfindungsgemäß ebenso wie die reinen Verbindungen eingesetzt werden.

Erfindungsgemäß einsetzbar neben den bevorzugten Dicarbonsäuren gemäß Formel (N-I) sind auch solche Dicarbonsäuren, die sich von den Verbindungen gemäß Formel (N-I) durch 1 bis 3 Methyl- oder Ethyl-Substituenten am Cyclohexylring unterscheiden oder aus diesen Verbindungen formal durch Anlagerung von einem Molekül Wasser an die Doppelbildung des Cyclohexenrings gebildet werden.

Als erfindungsgemäß besonders vorteilhaft hat sich die Dicarbonsäure(-mischung) erwiesen, die durch Umsetzung von Linolsäure mit Acrylsäure entsteht. Es handelt sich dabei um eine Mischung aus 5- und 6-Carboxy-4-hexyl-2-cyclohexen-1-octansäure. Solche Verbindungen sind kommerziell unter den Bezeichnungen Westvaco Diacid® 1550 und Westvaco Diacid® 1595 (Hersteller: Westvaco) erhältlich.

Neben den zuvor beispielhaft aufgeführten erfindungsgemäßen kurzkettigen Carbonsäuren selbst können auch deren physiologisch verträgliche Salze erfindungsgemäß eingesetzt werden. Beispiele für solche Salze sind die Alkali-, Erdalkali-, Zinksalze sowie Ammoniumsalze, worunter im Rahmen der vorliegenden Anmeldung auch die Mono-, Di- und Trimethyl-, -ethyl- und -hydroxyethyl-Ammoniumsalze zu verstehen sind. Ganz besonders bevorzugt können im Rahmen der Erfindung jedoch mit alkalisch reagierenden Aminosäuren, wie beispielsweise Arginin, Lysin, Ornithin und Histidin, neutralisierte Säuren eingesetzt werden. Weiterhin kann es aus Formulierungsgründen bevorzugt sein, die Carbonsäure aus den wasserlöslichen Vertretern, insbesondere den wasserlöslichen Salzen, auszuwählen.

Weiterhin ist es erfindungsgemäß bevorzugt, Hydroxycarbonsäuren und hierbei wiederum insbesondere die Dihydroxy-, Trihydroxy- und Polyhydroxycarbonsäuren sowie die Dihydroxy-, Trihydroxy- und Polyhydroxy- di-, tri- und polycarbonsäuren einzusetzen. Hierbei hat sich gezeigt, daß neben den Hydroxycarbonsäuren auch die Hydroxycarbonsäureester sowie die Mischungen aus Hydroxycarbonsäuren und deren Estern als auch polymere Hydroxycarbonsäuren und deren Ester ganz besonders bevorzugt sein können. Bevorzugte Hydroxycarbonsäureester sind beispielsweise Vollester der Glycolsäure, Milchsäure, Äpfelsäure, Weinsäure oder Citronensäure. Weitere grundsätzlich geeignete Hydroxycarbonsäureester sind Ester der β-Hydroxypropionsäure, der Tartronsäure, der D-Gluconsäure, der Zuckersäure, der Schleimsäure oder der Glucuronsäure. Als Alkoholkomponente dieser Ester eignen sich primäre, lineare oder verzweigte aliphatische Alkohole mit 8 - 22 C-Atomen, also z.B. Fettalkohole oder synthetische Fettalkohole. Dabei sind die Ester von C12-C15-Fettalkoholen besonders bevorzugt. Ester dieses Typs sind im Handel erhältlich, z.B. unter dem Warenzeichen Cosmacol® der EniChem, Augusta Industriale. Besonders bevorzugte Polyhydroxypolycarbonsäuren sind Polymilchsäure und Polyweinsäure sowie deren Ester.

Weiterhin sind als konditionierende Wirkstoffe geeignet Silikonöle und Silikon-Gums, insbesondere Dialkyl-.und Alkylarylsiloxane, wie beispielsweise Dimethylpolysiloxan und Methylphenylpolysiloxan, sowie deren alkoxylierte und quaternierte Analoga. Beispiele für solche Silikone sind die von Dow Corning unter den Bezeichnungen DC 190, DC 200 und DC 1401 vertriebenen Produkte sowie das Handelsprodukt Fancorsil® LIM-1.

Erfindungsgemäß als konditionierende Wirkstoffe ebenfalls geeignet sind katiohische Silikonöle wie beispielsweise die im Handel erhältlichen Produkte Q2-7224 (Hersteller: Dow Corning; ein stabilisiertes Trimethylsilylamodimethicon), Dow Corning® 939 Emulsion (enthaltend ein hydroxyl-amino-modifiziertes Silicon, das auch als Amodimethicone bezeichnet wird), SM-2059 (Hersteller: General Electric), SLM-55067 (Hersteller: Wacker) sowie Abil®-Quat 3270 und 3272 (Hersteller: Th. Goldschmidt; diquaternäre Polydimethylsiloxane, Quaternium-80). Ein geeignetes anionisches Silikonöl ist das Produkt Dow Corning®1784.

Weitere Wirk-, Hilfs- und Zusatzstoffe sind beispielsweise
- Verdickungsmittel wie Agar-Agar, Guar-Gum, Alginate, Xanthan-Gum, Gummi arabicum, Karaya-Gummi, Johannisbrotkernmehl, Leinsamengummen, Dextrane, Cellulose-Derivate, z. B. Methylcellulose, Hydroxyalkylcellulose und Carboxymethylcellulose, Stärke-Fraktionen und Derivate wie Amylose, Amylopektin und Dextrine, Tone wie z. B. Bentonit oder vollsynthetische Hydrokolloide wie z. B. Polyvinylalkohol,
- haarkonditionierende Verbindungen wie Phospholipide, beispielsweise Sojalecithin, Ei-Lecitin und Kephaline, sowie Silikonöle,
- Parfümöle, Dimethylisosorbid und Cyclodextrine,
- Lösungsmittel und -vermittler wie Ethanol, Isopropanol, Ethylenglykol, Propylenglykol, Glycerin und Diethylenglykol,
- faserstrukturverbessernde Wirkstoffe, insbesondere Mono-, Di- und Oligosaccharide, wie beispielsweise Glucose, Galactose, Fructose, Fruchtzucker und Lactose,
- konditionierende Wirkstoffe wie Paraffinöle, pflanzliche Öle, z. B. Sonnenblumenöl, Orangenöl, Mandelöl, Weizenkeimöl und Pfirsichkernöl sowie quaternierte Amine wie Methyl-1-alkylamidoethyl-2-alkylimidazolinium-methosulfat,
- Entschäumer wie Silikone,
- Farbstoffe zum Anfärben des Mittels,
- Antischuppenwirkstoffe wie Piroctone Olamine, Zink Omadine und Climbazol,
- Wirkstoffe wie Allantoin und Bisabolol, Cholesterin,
- Konsistenzgeber wie Zuckerester, Polyolester oder Polyolalkylether,
- Fette und Wachse wie Walrat, Bienenwachs, Montanwachs und Paraffine,
- Fettsäurealkanolamide,
- Komplexbildner wie EDTA, NTA, β-Alanindiessigsäure und Phosphonsäuren,
- Quell- und Penetrationsstoffe wie primäre, sekundäre und tertiäre Phosphate,
- Trübungsmittel wie Latex, Styrol/PVP- und Styrol/Acrylamid-Copolymere,
- Perlglanzmittel wie Ethylenglykolmono- und -distearat sowie PEG-3-distearat,
- Pigmente,
- Treibmittel wie Propan-Butan-Gemische, N₂O, Dimethylether, CO₂ und Luft,
- Antioxidantien.

Bezüglich weiterer fakultativer Komponenten sowie die eingesetzten Mengen dieser Komponenten wird ausdrücklich auf die dem Fachmann bekannten einschlägigen Handbücher, z. B. die Monographie von K. H. Schrader, Grundlagen und Rezepturen der Kosmetika, 2. Auflage, Hüthig Buch Verlag Heidelberg, 1989, verwiesen.

Ein zweiter Gegenstand der Erfindung ist ein Verfahren zur milden Reinigung und schonenden Pflege von Haut und Haar bei dem die erfindungsgemäße Zusammensetzung auf die Haut und/oder das Haar aufgetragen, verteilt und mit Wasser wieder abgespült wird.

Ein dritter Gegenstand der Erfindung ist die Verwendung der erfindungsgemäßen Zusammensetzung zur milden Reinigung und schonenden Pflege von Haut und Haar.

Die folgenden Beispiele sollen die Erfindung näher erläutern, ohne ihn darauf zu beschränken - alle Gewichtsangaben beziehen sich auf Gew.%:

| Duschöl mit verbesserter Hautverträglichkeit : | |
|---|---|
| 1,2-Propylenglycol | 1,00 |
| Glycerin 86% DAB pflanzl. | 10,00 |
| Natriumlaurethsulfat | 12,00 |
| Polyethylenglycol 400 | 5,00 |
| Aristoflex® PEA | 2,00 |
| Sorbit 70% DAB | 2,00 |
| Isopropylmyristat | 2,00 |
| Monomuls® 90-O-18 | 1,00 |
| Tegosoft® GMC 6 | 1,00 |
| Cetiol® HE | 5,00 |
| Plantaren® 2000 CS/UP | 4,50 |
| Akypo® RLM 100 | 2,70 |
| Natriumbenzoat | 0,50 |
| Zitronensäure | 0,20 |
| Parfümöl | 0,85 |
| Farbstoff, Wasser | ad 100 |

## Patentansprüche

1. Wässrige Körperreinigungs- und Pflegezusammensetzungen, enthaltend
- maximal 35 Gew.-% einer milden Tensidmischung, bestehend aus
(A) mindestens einem anionischen Tensid vom Typ der Fettalkoholpolyglycolethersulfate,
(B) mindestens einem Tensid auf Zuckerbasis und
(C) mindestens einem Co-Tensid, ausgewählt aus der Gruppe, die gebildet wird aus Sulfosuccinaten, Sulfobernsteinsäureestern, α-Olefinsulfonaten, Acyltauriden und Fettalkohol-Ethercarbonsäuren, und
- 2 bis 25 Gew.-% eines Ölkörpers,
**dadurch gekennzeichnet, dass** die Tenside in einem Gewichtsverhältnis von (A) : (B) : (C) = 10 : (0,5 - 5) : (0,5 - 5) vorliegen.

2. Wässrige Körperreinigungs- und Pflegezusammensetzungen nach Anspruch 1, **dadurch gekennzeichnet, dass** sie als Komponente (A) Fettalkoholpolyglycolethersulfate der Formel (I)
R¹O-(CH₂CH₂O)ₙSO₃X (I)
in der R¹ für einen linearen oder verzweigten Alkyl- und/oder Alkenylrest mit 12 bis 18 Kohlenstoffatomen, n für Zahlen von 1 bis 5 und X für Alkali oder Ammonium stehen, enthalten.

3. Wässrige Körperreinigungs- und Pflegezusammensetzungen nach einem der Ansprüche 1 oder 2, **dadurch gekennzeichnet, dass** der Gehalt der Komponente (A) in der Tensidmischung 0,1 bis 80 Gew.-% beträgt.

4. Wässrige Körperreinigungs- und Pflegezusammensetzungen nach einem der Ansprüche 1 bis 3, **dadurch gekennzeichnet, dass** sie als Komponente (B) Alkyl- und/oder Alkenyloligoglycoside der Formel (II)
R²O-[G]ₘ (II)
in der R² für einen Alkyl- und/oder Alkenylrest mit 4 bis 22 Kohlenstoffatomen, G für einen Zuckerrest mit 5 bis 6 Kohlenstoffatomen und m für Zahlen von 1 bis 10 steht.

5. Wässrige Körperreinigungs- und Pflegezusammensetzungen nach einem der Ansprüche 1 bis 4, **dadurch gekennzeichnet, dass** der Gehalt der Komponente (B) in der Tensidmischung 0,1 bis 30 Gew.-% beträgt.

6. Wässrige Körperreinigungs- und Pflegezusammensetzungen nach einem der Ansprüche 1 bis 5, **dadurch gekennzeichnet, dass** sie als Co-Tenside (C) Fettalkohol-Ethercarbonsäuren der Formel (III)
CH₃(CH₂)ₚ(OCH₂CH₂)_{q}OCH₂(CH₂)ᵣCOOH (III)
sowie physiologisch verträgliche Salze dieser Verbindungen, in denen p steht für Zahlen von 6 bis 30, q steht für Zahlen von 1 bis 30 und r steht für Zahlen von 0 bis 22.

7. Wässrige Körperreinigungs- und Pflegezusammensetzungen nach einem der Ansprüche 1 bis 6, **dadurch gekennzeichnet, dass** der Gehalt der Komponente (C) in der Tensidmischung 0,1 bis 30 Gew.-% beträgt.

8. Wässrige Körperreinigungs- und Pflegezusammensetzungen nach einem der Ansprüche 1 bis 7, **dadurch gekennzeichnet, dass** als Ölkörper Esteröle, Fettsäureglyceride und/oder ethoxylierte Fettsäureglyceride enthalten sind.

9. Wässrige Körperreinigungs- und Pflegezusammensetzungen nach einem der Ansprüche 1 bis 8, **dadurch gekennzeichnet, dass** die Esteröle sich aus C₆ - C₃₀ - Fettsäuren mit C₂ - C₃₀ - Fettalkoholen zusammensetzen.

10. Wässrige Körperreinigungs- und Pflegezusammensetzung nach einem der Ansprüche 1 bis 8, **dadurch gekennzeichnet, dass** sich die Fettsäureglyceride aus Mono,- Di- und Trifettsäureestern von gesättigten und/oder ungesättigten linearen und/oder verzweigten C₆ - C₃₀ - Fettsäuren mit Glycerin zusammensetzen.

11. Wässrige Körperreinigungs- und Pflegezusammensetzungen nach einem der Ansprüche 1 bis 8, **dadurch gekennzeichnet, dass** sich die ethoxylierten Fettsäureglyceride aus 1-30-fach ethoxylierten Mono,- Di- und Trifettsäureestern von gesättigten und/oder ungesättigten linearen und/oder verzweigten C₆ - C₃₀ - Fettsäuren mit Glycerin zusammensetzen.

12. Wässrige Körperreinigungs- und Pflegezusammensetzungen nach einem der Ansprüche 1 bis 11, **dadurch gekennzeichnet, dass** sie 4 bis 25 Gew.-%, bezogen auf die gesamte Zusammensetzung, des Ölkörpers enthalten.

13. Wässrige Körperreinigungs- und Pflegezusammensetzungen nach einem der Ansprüche 1 bis 12, **dadurch gekennzeichnet, dass** sie 6 bis Gew.-20%, bezogen auf die gesamte Zusammensetzung, des Ölkörpers enthalten.

14. Wässrige Körperreinigungs- und Pflegezusammensetzungen nach einem der Ansprüche 1 bis 13, **dadurch gekennzeichnet, dass** weiterhin mindestens ein kationisches und/oder nichtionisches Polymer enthalten ist.

15. Wässrige Körperreinigungs- und Pflegezusammensetzungen nach einem der Ansprüche 1 bis 14, **dadurch gekennzeichnet, dass** die kationischen und nichtionischen Polymere ausgewählt sind aus der Gruppe die gebildet wird aus kationischen Cellulosederivaten, Homopolymeren des Dimethyldiallylammoniumchlorids oder Copolymeren der Acrylamid mit Dimethyldiallylammoniumchlorid, kationischen Guar-Gummen, Vinylpyrrolidon/Vinylester-Copolymeren, Celluloseethern, Polyvinylpyrrolidonen, Siloxanen und Copolymeren des Polyethylens oder Polypropylens mit Terephthalsäure.

16. Wässrige Körperreinigungs- und Pflegezusammensetzungen nach einem der Ansprüche 1 bis 15, **dadurch gekennzeichnet, dass** die kationischen und nichtionischen Polymere bevorzugt in Mengen von 0,01 bis 10 Gew%, bezogen auf die gesamte Zusammensetzung, enthalten sind.

17. Wässrige Körperreinigungs- und Pflegezusammensetzungen nach einem der Ansprüche 1 bis 16, **dadurch gekennzeichnet, dass** sie weiterhin einen oder mehrere Vertreter aus der Gruppe der Polyole enthalten.

18. Wässrige Körperreinigungs- und Pflegezusammensetzungen nach Anspruch 17, **dadurch gekennzeichnet, dass** der Polyolgehalt 0,01 bis 35 Gew.- %, bezogen auf die gesamte Zusammensetzung, beträgt.

19. Wässrige Körperreinigungs- und Pflegezusammensetzungen nach einem der Ansprüche 17 oder 18, **dadurch gekennzeichnet, dass** der Polyolgehalt 1 bis 20 Gew.- %, bezogen auf die gesamte Zusammensetzung, beträgt.

20. Verfahren zur milden Reinigung und schonenden Pflege von Haut und Haar, **dadurch gekennzeichnet, dass** ein wässriges Mittel nach einem der Ansprüche 1 bis 19 auf die Haut und/oder das Haar aufgetragen, verteilt und mit Wasser wieder abgespült wird.

21. Verwendung einer wässrigen Körperreinigungs- und Pflegezusammensetzung nach einem der Ansprüche 1 bis 19 zur milden Reinigung und schonenden Pflege von Haut und Haar.

## Claims

1. Aqueous body-cleansing and care compositions comprising
- at most 35% by weight of a mild surfactant mixture consisting of
(A) at least one anionic surfactant of the fatty alcohol polyglycol ether sulphate type,
(B) at least one sugar-based surfactant and
(C) at least one cosurfactant chosen from the group formed from sulphosuccinates, sulphosuccinic esters, α-olefinsulphonates, acyl taurides and fatty alcohol ether carboxylic acids, and
- 2 to 25% by weight of an oil body,
**characterized in that** the surfactants are present in a weight ratio of (A) : (B) : (C) = 10: (0.5-5) : (0.5-5).

2. Aqueous body-cleansing and care compositions according to Claim 1, **characterized in that** they comprise, as component (A), fatty alcohol polyglycol ether sulphates of the formula (I)
R¹O-(CH₂CH₂O)ₙSO₃X (I)
in which R¹ is a linear or branched alkyl and/or alkenyl radical having 12 to 18 carbon atoms, n is numbers from 1 to 5 and X is alkali metal or ammonium.

3. Aqueous body-cleansing and care compositions according to one of Claims 1 and 2, **characterized in that** the content of component (A) in the surfactant mixture is 0.1 to 80% by weight.

4. Aqueous body-cleansing and care compositions according to one of Claims 1 to 3, **characterized in that** they comprise, as component (B), alkyl and/or alkenyl oligoglycosides of the formula (II)
R²O-[G]ₘ (II)
in which R² is an alkyl and/or alkenyl radical having 4 to 22 carbon atoms, G is a sugar radical having 5 to 6 carbon atoms and m is numbers from 1 to 10.

5. Aqueous body-cleansing and care compositions according to one of Claims 1 to 4, **characterized in that** the content of component (B) in the surfactant mixture is 0.1 to 30% by weight.

6. Aqueous body-cleansing and care compositions according to one of Claims 1 to 5, **characterized in that** they comprise, as cosurfactants (C), fatty alcohol ether carboxylic acids of the formula (III)
CH₃(CH₂)ₚ(OCH₂CH₂)_{q}OCH₂(CH₂)ᵣCOOH (III)
and physiologically compatible salts of these compounds, in which p is numbers from 6 to 30, q is numbers from 1 to 30 and r is numbers from 0 to 22.

7. Aqueous body-cleansing and care compositions according to one of Claims 1 to 6, **characterized in that** the content of component (C) in the surfactant mixture is 0.1 to 30% by weight.

8. Aqueous body-cleansing and care compositions according to one of Claims 1 to 7, **characterized in that** the oil bodies present are ester oils, fatty acid glycerides and/or ethoxylated fatty acid glycerides.

9. Aqueous body-cleansing and care compositions according to one of Claims 1 to 8, **characterized in that** the ester oils are composed of C₆-C₃₀-fatty acids with C₂-C₃₀-fatty alcohols.

10. Aqueous body-cleansing and care composition according to one of Claims 1 to 8, **characterized in that** the fatty acid glycerides are composed of mono-, di- and trifatty acid esters of saturated and/or unsaturated linear and/or branched C₆-C₃₀-fatty acids with glycerol.

11. Aqueous body-cleansing and care compositions according to one of Claims 1 to 8, **characterized in that** the ethoxylated fatty acid glycerides are composed of 1-30-fold ethoxylated mono-, di- and trifatty acid esters of saturated and/or unsaturated linear and/or branched C₆-C₃₀-fatty acids with glycerol.

12. Aqueous body-cleansing and care compositions according to one of Claims 1 to 11, **characterized in that** they comprise 4 to 25% by weight, based on the total composition, of the oil body.

13. Aqueous body-cleansing and care compositions according to one of Claims 1 to 12, **characterized in that** they comprise 6 to 20% by weight, based on the total composition, of the oil body.

14. Aqueous body-cleansing and care compositions according to one of Claims 1 to 13, **characterized in that** at least one cationic and/or nonionic polymer is also present.

15. Aqueous body-cleansing and care compositions according to one of Claims 1 to 14, **characterized in that** the cationic and nonionic polymers are chosen from the group which is formed from cationic cellulose derivatives, homopolymers of dimethyldiallylammonium chloride or copolymers of acrylamide with dimethyldiallylammonium chloride, cationic guar gums, vinylpyrrolidone/vinyl ester copolymers, cellulose ethers, polyvinylpyrrolidones, siloxanes and copolymers of polyethylene or polypropylene with terephthalic acid.

16. Aqueous body-cleansing and care compositions according to one of Claims 1 to 15, **characterized in that** the cationic and nonionic polymers are preferably present in amounts of from 0.01 to 10% by weight, based on the total composition.

17. Aqueous body-cleansing and care compositions according to one of Claims 1 to 16, **characterized in that** they also comprise one or more representatives from the group of polyols.

18. Aqueous body-cleansing and care compositions according to Claim 17, **characterized in that** the polyol content is 0.01 to 35% by weight, based on the total composition.

19. Aqueous body-cleansing and care compositions according to one of Claims 17 and 18, **characterized in that** the polyol content is 1 to 20% by weight, based on the total composition.

20. Method for the mild cleansing and gentle care of skin and hair, **characterized in that** an aqueous composition according to one of Claims 1 to 19 is applied to the skin and/or the hair, distributed and washed off again with water.

21. Use of an aqueous body-cleansing and care composition according to one of Claims 1 to 19 for the mild cleansing and gentle care of skin and hair.

## Revendications

1. Compositions aqueuses de nettoyage et de soin pour le corps, contenant
- au maximum 35% en poids d'un mélange d'agents tensioactifs doux, constitué par
(A) au moins un agent tensioactif anionique du type des polyglycoléthersulfates d'alcool gras
(B) au moins un agent tensioactif à base de sucre et
(C) au moins un co-agent tensioactif, choisi dans le groupe formé par les sulfosuccinates, les esters de l'acide sulfosuccinique, les α-oléfinesulfonates, les acyltaurides et les acides éthercarboxyliques d'alcools gras et
- 2 à 25% en poids d'un corps huileux,
**caractérisé en ce que** les agents tensioactifs se trouvent dans un rapport pondéral (A) : (B) : (C) = 10 : (0,5 - 5) : (0,5 - 5).

2. Compositions aqueuses de nettoyage et de soin pour le corps selon la revendication 1, **caractérisées en ce qu'**elles contiennent, comme composant (A) des polyglycoléthersulfates d'alcool gras de formule (I)
R¹O-(CH₂CH₂O)ₙSO₃X (I)
dans laquelle R¹ représente un radical alkyle et/ou alcényle linéaire ou ramifié comprenant 12 à 18 atomes de carbone, n représente des nombres de 1 à 5 et X représente un alcalin ou ammonium.

3. Compositions aqueuses de nettoyage et de soin pour le corps selon l'une quelconque des revendications 1 ou 2, **caractérisées en ce que** la teneur en composant (A) dans le mélange d'agents tensioactifs est de 0,1 à 80% en poids.

4. Compositions aqueuses de nettoyage et de soin pour le corps selon l'une quelconque des revendications 1 à 3, **caractérisées en ce qu'**elles contiennent comme composant (B) des alkyloligoglycosides et/ou des alcényloligoglycosides de formule (II)
R²O-[G]ₘ (II)
dans laquelle R² représente un radical alkyle et/ou alcényle comprenant 4 à 22 atomes de carbone, G représente un radical de sucre comprenant 5 à 6 atomes de carbone et m représente les nombres de 1 à 10.

5. Compositions aqueuses de nettoyage et de soin pour le corps selon l'une quelconque des revendications 1 à 4, **caractérisées en ce que** la teneur en composant (B) dans le mélange d'agents tensioactifs est de 0,1 à 30% en poids.

6. Compositions aqueuses de nettoyage et de soin pour le corps selon l'une quelconque des revendications 1 à 5, **caractérisées en ce qu'**elles contiennent, comme co-agents tensioactifs (C) des acides éthercarboxyliques d'alcool gras de formule (III)
CH₃(CH₂)ₚ(OCH₂CH₂)_{g}OCH₂(CH₂)ᵣCOOH (III)
ainsi que les sels physiologiquement acceptables de ces composés, dans laquelle p représente des nombres de 6 à 30, q représente des nombres de 1 à 30 et r représente des nombres de 0 à 22.

7. Compositions aqueuses de nettoyage et de soin pour le corps selon l'une quelconque des revendications 1 à 6, **caractérisées en ce que** la teneur en composant (C) dans le mélange d'agents tensioactifs est de 0,1 à 30% en poids.

8. Compositions aqueuses de nettoyage et de soin pour le corps selon l'une quelconque des revendications 1 à 7, **caractérisées en ce qu'**elles contiennent, comme corps gras, des huiles d'ester, des glycérides d'acide gras et/ou des glycérides éthoxylés d'acide gras.

9. Compositions aqueuses de nettoyage et de soin pour le corps selon l'une quelconque des revendications 1 à 8, **caractérisées en ce que** les huiles d'ester sont composées par des acides gras en C₆ à C₃₀ avec des alcools gras en C₂ à C₃₀.

10. Composition aqueuse de nettoyage et de soin pour le corps selon l'une quelconque des revendications 1 à 8, **caractérisée en ce que** les glycérides d'acide gras sont composés par des monoesters, des diesters ou des triesters d'acides gras saturés et/ou insaturés, linéaires et/ou ramifiés en C₆ à C₃₀ avec du glycérol.

11. Compositions aqueuses de nettoyage et de soin pour le corps selon l'une quelconque des revendications 1 à 8, **caractérisées en ce que** les glycérides d'acide gras éthoxylés sont composés par des monoesters, des diesters et des triesters éthoxylés 1 à 30 fois d'acides gras saturés et/ou insaturés, linéaires et/ou ramifiés en C₆ à C₃₀ avec du glycérol.

12. Compositions aqueuses de nettoyage et de soin pour le corps selon l'une quelconque des revendications 1 à 11, **caractérisées en ce qu'**elles contiennent 4 à 25% en poids, par rapport à la totalité de la composition, de corps huileux.

13. Compositions aqueuses de nettoyage et de soin pour le corps selon l'une quelconque des revendications 1 à 12, **caractérisées en ce qu'**elles contiennent 6 à 20% en poids, par rapport à la totalité de la composition, de corps huileux.

14. Compositions aqueuses de nettoyage et de soin pour le corps selon l'une quelconque des revendications 1 à 13, **caractérisées en ce qu'**elles contiennent en outre au moins un polymère cationique et/ou non ionique.

15. Compositions aqueuses de nettoyage et de soin pour le corps selon l'une quelconque des revendications 1 à 14, **caractérisées en ce que** les polymères cationiques et non ioniques sont choisis dans le groupe constitué par les dérivés cationiques de la cellulose, les homopolymères du chlorure de diméthyldiallylammonium ou les copolymères de l'acrylamide avec du chlorure de diméthyldiallylammonium, les gommes guar cationiques, les copolymères de vinylpyrrolidone/ester de vinyle, les éthers de cellulose, les polyvinylpyrrolidones, les siloxanes et les copolymères du polyéthylène ou du polypropylène avec de l'acide téréphtalique.

16. Compositions aqueuses de nettoyage et de soin pour le corps selon l'une quelconque des revendications 1 à 15, **caractérisées en ce que** les polymères cationiques et non ioniques sont de préférence contenus en des quantités de 0,01 à 10% en poids, par rapport à la totalité de la composition.

17. Compositions aqueuses de nettoyage et de soin pour le corps selon l'une quelconque des revendications 1 à 16, **caractérisées en ce qu'**elles contiennent en outre un ou plusieurs représentants du groupe des polyols.

18. Compositions aqueuses de nettoyage et de soin pour le corps selon la revendication 17, **caractérisées en ce que** la teneur en polyol est de 0,01 à 35% en poids par rapport à la totalité de la composition.

19. Compositions aqueuses de nettoyage et de soin pour le corps selon l'une quelconque des revendications 17 ou 18, **caractérisées en ce que** la teneur en polyol est de 1 à 20% en poids par rapport à la totalité de la composition.

20. Procédé pour le nettoyage doux et le soin ménagé de la peau et des cheveux, **caractérisé en ce qu'**un agent aqueux selon l'une quelconque des revendications 1 à 19 est appliqué et réparti sur la peau et/ou les cheveux et est à nouveau éliminé par rinçage avec de l'eau.

21. Utilisation d'une composition aqueuse de nettoyage et de soin pour le corps selon l'une quelconque des revendications 1 à 19 pour le nettoyage doux et le soin ménagé de la peau et des cheveux.
